# EUROPEAN PATENT SPECIFICATION

(11) **EP 3 361 973 B1**
(45) Date of publication and mention of the grant of the patent: **23.09.2020**
(21) Application number: 16791322.7
(22) Date of filing: 11.10.2016
(51) Int. Cl.: A61B 17/44, A61B 90/00, A61B 17/00, A61B 34/20, A61B 17/30

(54) **OBSTETRIC EXTRACTION**
OBSTETRISCHE EXTRAKTION
EXTRACTION OBSTÉTRIQUE

(30) Priority: 13.10.2015 DE 102015219840
(43) Date of publication of application: 22.08.2018
(73) Proprietor: Genit Innovations AB, 112 39 Stockholm (SE)
(72) Inventor: YOUSAF, Khurram, 151 60 Södertälje (SE); STURM, Dennis, 23552 Lübeck (DE); AJNE, Gunilla, 141 91 Huddinge (SE); WESTGREN, Magnus, 112 64 Stockholm (SE)
(74) Representative: Maikowski & Ninnemann Patentanwälte Partnerschaft mbB
(86) International application number: PCT/EP2016/074334
(87) International publication number: WO 2017/064057

(56) References cited:
- WO-A1-2011/032065
- CN-A- 102 789 723
- US-A1- 2003 114 779
- US-A1- 2005 014 115

## Description

### TECHNICAL FIELD

This specification refers to embodiments of an obstetric extraction apparatus and to embodiments of a method of operating an obstetric extraction apparatus. In particular, this specification refers to embodiments of an obstetric vacuum extraction apparatus and to embodiments of a corresponding operating method, wherein a progress monitoring and an outcome prediction is carried out.

### BACKGROUND

A certain percentage of all vaginal deliveries comprises applying a vacuum extraction. A vacuum extraction is a well-accepted procedure for vaginal delivery in which usually a suction cup docketed to the head of a fetus is used to pull the fetus out of the birth canal.

The obstetric extraction procedure is sometimes executed with little control and very subjectively. If the vacuum extraction is executed wrongly it can lead to permanent damage or even fatal consequences for the child and/or the birthing mother.

For example, an obstetric extraction apparatus is known from US 2005/0045098 A1 and from US 7,291,156 B1.

CN 102 789 723 A describes a trainer for simulating operation of obstetric forceps. This document proposes the following teaching: an external force acts against a fetal head through the obstetric forceps; position and attitude parameters of the obstetric forceps and the fetal head are transmitted to a computer through a three-dimensional space track locator; clamping force from the obstetric forceps and traction force which are exerted to the fetal head are transmitted to a lower computer system through a clamping force sensor and a traction force sensor respectively; data is sent to the computer by the lower computer system; position and attitude of each 3D (three-dimensional) model in a three-dimensional scene is updated by a software system which runs over a high-performance computer platform according to the collected position and attitude data, and actual operation acts in a three-dimensional scene to realize synchronous representation in real time; a fetal head motion control command is generated by the system according to stress data, and the command is sent to the lower computer system; and a fetal head drive device is controlled by the lower computer system to drive the fetal head to move.

### SUMMARY

The invention is defined in the appended claims.

According to an embodiment, an obstetric extraction apparatus, comprises: an extractor having a distal element and a proximal element coupled thereto, the distal element being configured to be docketed to a fetal head, and the proximal element being configured to be gripped by hand; a beacon configured to be attached stationary and separately from the extractor; a sensor mechanically coupled to the extractor and configured to provide a force signal representative of an extraction force applied to the fetal head; and a processor communicatively coupled to at least the extractor, wherein the processor is configured to receive the force signal and to carry out a value-based determination of a progress parameter set indicative of at least one of a distance between the extractor and the beacon and a motion of the extractor relative to the beacon. The user feedback device comprises a haptic feedback unit included in the proximal element.

According to an example disclosed herein, a method of operating an obstetric extraction apparatus comprises: carrying out a value-based determination of a force parameter set indicative of an extraction force applied to the fetal head by means of an extractor of the apparatus; carrying out a value-based determination of a progress parameter set indicative of at least one of a distance between the extractor and a beacon attached stationary and separately from the extractor and a motion of the extractor relative to the beacon; comparing at least one of the force parameter set and the progress parameter set with each of a plurality of data sets stored in storage means of the apparatus, each data set being indicative of a force-pattern and/or progress-pattern recorded for a previous real or virtual obstetric extraction; and controlling a user feedback device of the apparatus in dependence of the comparison.

Those skilled in the art will recognize additional features and advantages upon reading the following detailed description, and upon viewing the accompanying drawings.

### BRIEF DESCRIPTION OF THE DRAWINGS

The parts in the figures are not necessarily to scale, instead emphasis being placed upon illustrating principles of the disclosure. Moreover, in the figures, like reference numerals designate corresponding parts. In the drawings:
Fig. 1A schematically illustrates aspects of an obstetric extraction apparatus according to one or more embodiments;
Fig. 1B schematically illustrates aspects of an obstetric extraction apparatus according to one or more embodiments;
Fig. 1C schematically illustrates a method of operating an obstetric extraction apparatus according to one or more embodiments;
Fig. 1D schematically illustrates aspects of an obstetric extraction apparatus according to one or more embodiments;
Fig. 2 schematically illustrates aspects of a method of operating an obstetric extraction apparatus according to one or more embodiments;
Fig. 3 schematically illustrates aspects of an extractor of an obstetric extraction apparatus according to one or more embodiments;
Fig. 4 schematically illustrates aspects of an extractor of an obstetric extraction apparatus according to one or more embodiments;
Fig. 5a-c schematically illustrate further aspects of an extractor of an obstetric extraction apparatus according to one or more embodiments;
Fig. 6-7 schematically illustrates yet further aspects of an extractor of an obstetric extraction apparatus according to one or more embodiments;
Fig. 8a-c schematically illustrate a sensor element of an obstetric extraction apparatus according to one or more embodiments;
Fig. 9 schematically illustrates an aspect of a method of operating an obstetric extraction apparatus according to one or more embodiments;
Fig. 10-11 schematically illustrate further aspects of a method of operating an obstetric extraction apparatus according to one or more embodiments;
Fig. 12 schematically illustrates a further aspect of an extractor of an obstetric extraction apparatus according to one or more embodiments;
Fig. 13-14 schematically illustrate further aspects of a method of operating an obstetric extraction apparatus according to one or more embodiments;
Fig. 15a-b schematically illustrate a leg module of an obstetric extraction apparatus according to one or more embodiments;
Fig. 16a-c schematically illustrate a distal element of an obstetric extraction apparatus according to one or more embodiments;
Fig. 17-18 schematically illustrate further aspects of a method of operating an obstetric extraction apparatus according to one or more embodiments;
Fig. 19a-20c schematically illustrate aspects of an extractor of an obstetric extraction apparatus according to one or more embodiments;
Fig. 21 schematically illustrates a further aspect of a method of operating an obstetric extraction apparatus according to one or more embodiments;
Fig. 22-24b schematically illustrate aspects of an extractor of an obstetric extraction apparatus according to one or more embodiments;
Fig. 25-26 schematically illustrate further aspects of a method of operating an obstetric extraction apparatus according to one or more embodiments; and
Fig. 27 schematically illustrates aspects of a user feedback device of an obstetric extraction apparatus according to one or more embodiments.

### DETAILED DESCRIPTION

In the following detailed description, reference is made to the accompanying drawings which form a part hereof and in which are shown by way of illustration specific embodiments in which the disclosure may be practiced.

In this regard, directional terminology, such as "top", "bottom", "below", "front", "behind", "back", "leading", "trailing", "below", "above", "axial", "distal", "proximal" etc., may be used with reference to the orientation of the figures being described. Because parts of embodiments can be positioned in a number of different orientations, the directional terminology is used for purposes of illustration and is in no way limiting. It is to be understood that other embodiments may be utilized and structural or logical changes may be made without departing from the scope of the present disclosure. The following detailed description, therefore, is not to be taken in a limiting sense, and the scope of the present invention is defined by the appended claims.

Reference will now be made in detail to various embodiments, one or more examples of which are illustrated in the figures. Each example is provided by way of explanation, and is not meant as a limitation of the disclosure. For example, features illustrated or described as part of one embodiment can be used on or in conjunction with other embodiments to yield yet a further embodiment. It is intended that the present disclosure includes such modifications and variations. The examples are described using specific language which should not be construed as limiting the scope of the appended claims. The drawings are not scaled and are for illustrative purposes only. For clarity, the same elements or manufacturing steps have been designated by the same references in the different drawings if not stated otherwise.

Specific embodiments described in this specification pertain to, without being limited thereto, a vacuum extraction for vaginal delivery during which a suction cup coupled with the head of a fetus is used to pull the fetus out of the birth canal.

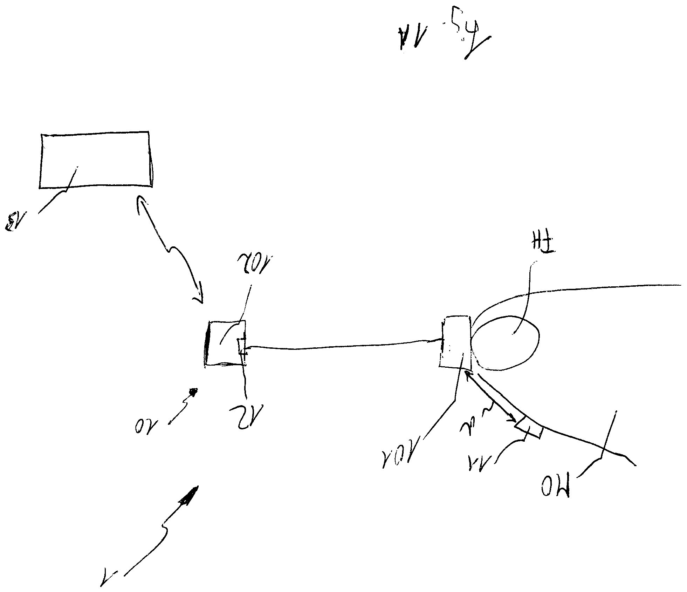

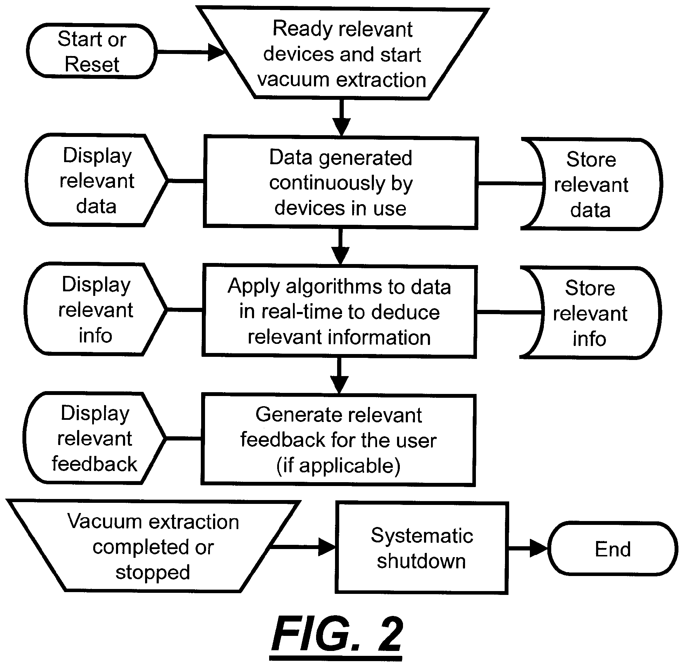

Fig. 1A schematically illustrates an obstetric extraction apparatus 1 in accordance with one or more embodiments. Fig. 2 presents an exemplarily process flowchart for the devices of an embodiment of the apparatus 1.

The obstetric extraction apparatus 1, in the following also simply referred to as "apparatus", comprises an extractor 10 that has a distal element 101 and a proximal element 102 coupled thereto. The distal element 101 can be configured to be docketed to a fetal head FH of the fetus (not illustrated). For example, the distal element 101 comprises a suction cup that can be attached to the fetal head FH, e.g., by generating a vacuum pressure between an inner surface of the suction cup and the fetal head FH. The proximal element 102 may be configured to be gripped by hand, e.g., by a doctor's (in the following also referred to as "user") hand that supervises the delivery. To this end, the proximal element 102 can comprise or be a handle bar, a grip or the alike.

To the extractor 10 is mechanically coupled a sensor 12 that is configured to provide a force signal representative of an extraction force applied to the fetal head FH. For example, said extraction force is applied by exerting a pull force onto the proximal element 102 and, thereby, onto the distal element 101 that is docketed to the fetal head FH. For example, the force signal is representative of each of a magnitude and a direction of the applied extraction force.

The apparatus 1 further comprises a processor 13 which may be arranged remotely from the extractor 10 but that is communicatively coupled to at least the extractor 10 and that is configured to receive the force signal generated by the sensor 12. The signal can be transferred from the sensor 12 in a wireless and/or in a wirebound manner. Thus, the processor 13 may be configured to determine the magnitude and/or the direction of the applied extraction force.

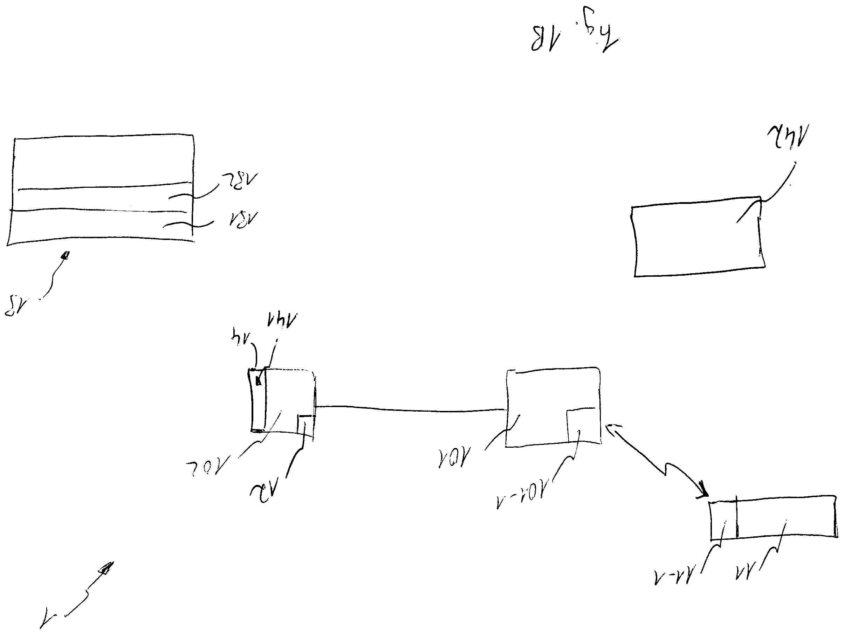

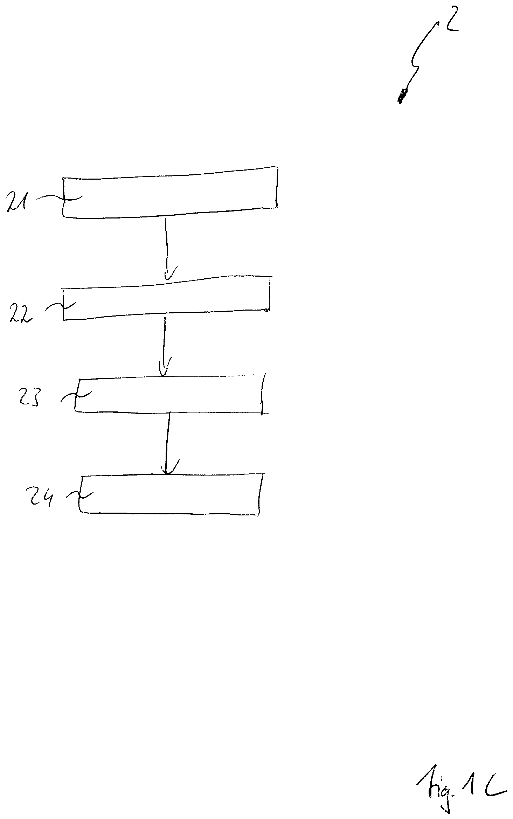

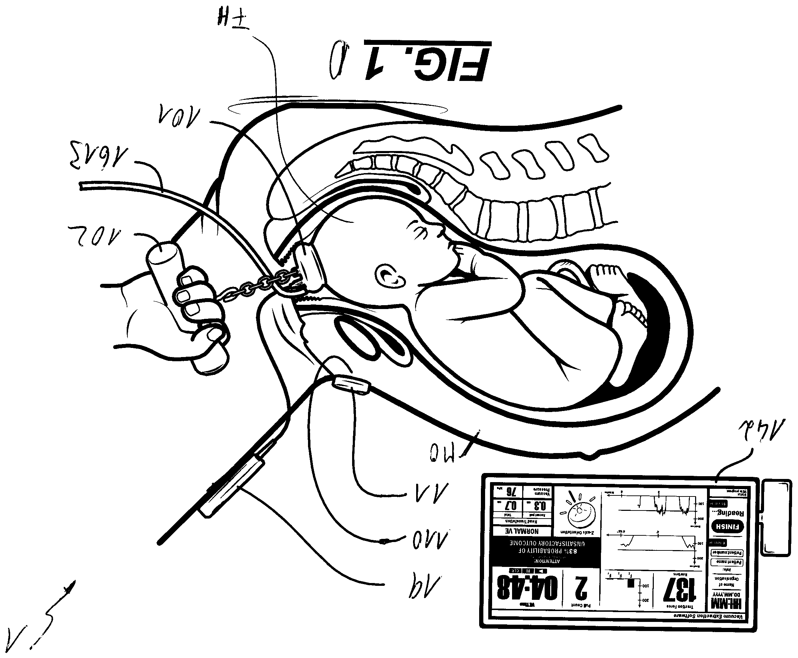

It shall be understood that the processor 13 may comprise one or more processor units (not illustrated in Fig. 1) that may be positioned in a distributed arrangement. For example, parts of the processor 13 may also be arranged within the extractor 10 itself, and other parts of the processor 13 may be arranged within, e.g., a computer, such as a desktop or laptop computer arranged in proximity to the birthing mother MO.

It shall further be understood that each signal transmission described herein can occur in a wireless or wirebound manner, if not explicitly stated otherwise.

The apparatus 1 may further comprise a beacon 11 that is configured to be attached stationary and is separately from the extractor 10, e.g., to the birthing mother MO. For example, the beacon is configured to be attached stationary with respect to the birth canal of the birthing mother MO. It should be understood that, within the present specification, the formulation "stationary" means that the beacon is arranged substantially stationary relative to, e.g., an element of the birthing mother. For example, the beacon 11 is clipped or glued to the mother's skin. For example, the beacon 11 may be configured for serving as a land mark regarding the fetal decent, i.e., the progress of the vaginal delivery.

In an embodiment, the processor 13 is configured to carry out a value-based determination of a progress parameter set that is indicative of at least one of a distance d between the extractor 10 and the beacon 11 and a motion of the extractor 10 relative to the beacon 11. The progress parameter set may include one or more progress parameters.

For example, the processor 13 may further be configured to carry out a value-based determination of a force parameter set in dependence of the force signal provided by the sensor 12. Also the force parameter set may include one or more force parameters. For example, a first force parameter may be indicative of an axial component of the applied extraction force, i.e., the force component that is directed in the direction parallel to the direction of a natural delivery (in the following also referred to as direction Z), and a second force parameter may be indicative of bending component of the applied extraction force, e.g., a force directed into a direction substantially perpendicular to the direction of the natural delivery. To this end, the sensor 12 may be a multi-axis force-measurement sensor and may comprise, e.g., 3-axis-force-measurement means. Thus, the force signal provided by the sensor 12 may include, e.g., a first force signal component indicative of said axial component of the applied extraction force and a second force signal component indicative of said bending component of the applied extraction force.

Regarding now in more detail the embodiment illustrated in Fig. 1B, the distal element 101 and the beacon 11 may comprise transceiver means 101-1 and 11-1 that are configured to transmit and receive a progress signal. Said transceiver means 101-1 and 11-1 may comprise or be constituted by a distance sensor and/or a motion sensor, e.g., an inertial sensor, exemplary embodiments of which are described in more detail below. Said sensors may each be configured to generate the progress signal and to provide the progress signal to the processor 13. For example, the processor 13 may be configured to carry out said value-based determination of the progress parameter set based on the progress signal(s) provided by one or more of the distance sensor and the motion sensor.

For example, by means of said transceiver means 101-1 and 11-1, the distal element 101 and the beacon 11 may exchange at least one of an electromagnetic signal, a radar signal and an ultrasound signal. Further, the processor 13 may be configured to carry out the value-based determination of the progress parameter set in dependence of the progress signal that has been exchanged by the distal element 101 and the beacon 11. For example, the exchanged progress signal may be indicative of the distance d between the beacon 11 and the distal element 101, i.e., indicative of the distance between the stationary attached beacon 11 and the suction cup docketed to the fetal head FH. Thereby, at least one of the distance d between the extractor 10 and the beacon 11 and the motion of the extractor 10 relative to the beacon 11 can be determined.

In accordance with a further embodiment, the extractor 10 may comprise means configured to provide an inertial signal, wherein the inertial signal may be indicative of an orientation of the distal element 101 relative to the proximal element 102, or, respectively, a movement of the proximal element 102 relative to the distal element 101. To this end, the extractor 10 may comprise, e.g., a gyroscope sensor (not illustrated). For example, said means configured to provide the inertial signal, e.g., the gyroscope sensor may be a part of the sensor 12 described above.

The apparatus 1 may further comprise a user feedback device 14 that is coupled to the processor 13. For example, the user feedback device 14 is communicatively coupled to the processor 13, e.g., in a manner that allows the processor 13 to control the user feedback device 14 and/or to receive user input via the user feedback device 14. Again, the coupling between the processor 13 and the user feedback device 14 maybe wirebound and/or wireless. For example, the processor 13 is configured to control the user feedback device 14 in dependence of at least of the force parameter set and the progress parameter set that has been determined by the processor 13.

In an embodiment, the user feedback device 14 comprises haptic feedback unit 141 that may be included in the proximal element 102. For example, the haptic feedback unit 141 comprise a vibrator. Thereby, the user exerting the extraction force to the proximal element 102 can be informed by the processor 13 about certain events. For example, the processor 13 may determine that the exerted extraction force is above or below a threshold value and/or or that the number of pull attempts has exceeded a predefined limit and may than cause the haptic feedback means 141 to vibrate, thereby informing the person exerting the extraction force about the corresponding event.

In accordance with a further embodiment, the user feedback device 14 may alternatively or additionally comprise a display unit 142 that may be arranged, e.g., in the field of vision of the user exerting the extraction force to the proximate element 102. Further, at least parts of the processor 13 and at least sections of the display unit 142 may be arranged within the same housing.

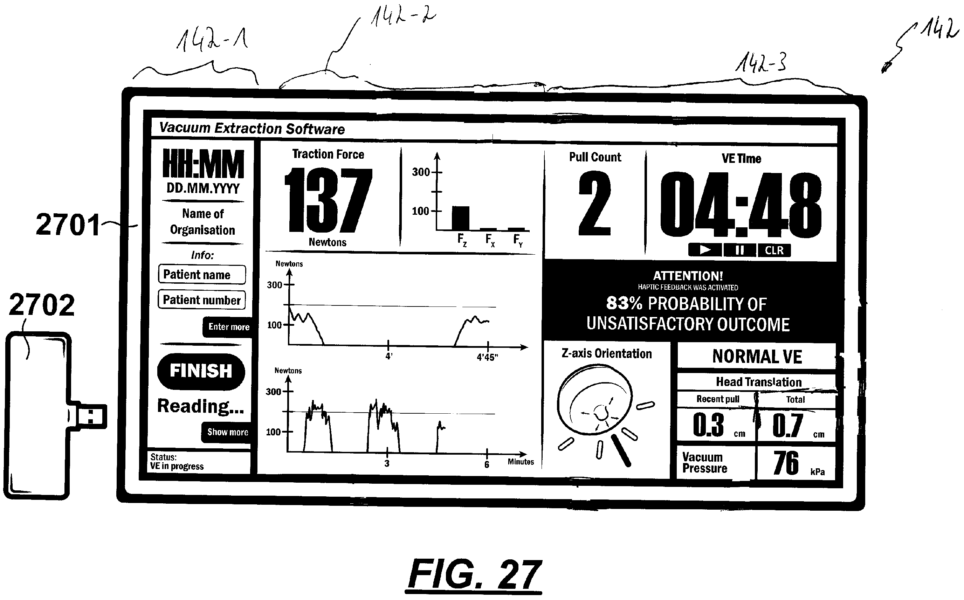

For example, the processor 13 is configured to control the display unit 142, e.g., based on the determined force parameter set and/or based on the determined progress parameter set. For example, the processor 13 induces the display unit 142 to display data being relevant to the current extraction procedure, e.g., by displaying at least one of the time, the number of pull attempts, the magnitude of the exerted extraction force, the angle of the exerted extraction force and further data. Further optional aspects of the display unit 142 and the control of the display unit 142 by means of the processor 13 will be explained in more detail with regards to Fig. 1D and Fig. 27.

In accordance with a further embodiment, the processor 13 may comprise storage means 131 that store a plurality of data sets, wherein each data set is indicative of a force-pattern and/or a progress-pattern that has been recorded for a previous real or virtual obstetric extraction.

Further, the processor 13 may be configured to compare at least one of the progress parameter set derived from the progress signal and the force parameter set derived from the force signal with each of the stored data sets. For example such comparison may allow the processor 13 to classify the current obstetric extraction as belonging to one of several categories such as "easy", "medium", or "heavy", and to control the user feedback device 14 in dependence of the classification. For example, the processor 13 is configured to inform the user exerting the extraction force to the proximal element 102 by means of the haptic feedback unit 141 and/or by means of the display unit 142 that the current extraction belongs to one of said categories.

Further, said comparison of the progress parameter set and/or the force parameter set with the force-progress-patterns stored in the storage means 131 may allow the processor 13 to predict the outcome of the current obstetric extraction and to control the user feedback device 14 based on this prediction. Said prediction may also depend on a classification as previously explained. Thus, in accordance with an embodiment, the processor 13 first carries out said classification of the current obstetric extraction and then, based on the classification, predicts the outcome of the current obstetric extraction in dependence of the determined progress parameter set and the determined force parameter set. The processor 13 may be configured to inform the person exerting the extraction force to the proximal element 102 by means of controlling the haptic feedback unit 141 and/or by controlling the display unit 142 such that corresponding prediction information is displayed.

In an embodiment, said comparison of the determined progress parameter set and/or the determined force parameter set with the stored force-progress-patterns may comprise applying at least one of a Principal-Component-Analysis (PCA), a neural network, data mining and/or another pattern recognition algorithm common to the skilled person by the processor 13.

In accordance with yet a further embodiment, the apparatus 1 may comprise a data logger 132, wherein the processor 13 can be configured to log, in the data logger 132, each of the determined progress parameter set and the determined force parameter set on a common time basis. For example, the data logger 132 may be a section of the data storage means 131 mentioned above. The processor 13 may be configured to convert the logged data into said data sets being indicative of a previous obstetric extraction. Thus, the processor 13 may be configured to carry out a learning process regarding at least one of said classification and said prediction.

Fig. 1C schematically illustrates a block diagram of a method 2 of operating an obstetric extraction apparatus, e.g., the obstetric extraction apparatus 1 that has been explained above with regards to Fig. 1A and Fig. 1B.

In a step 21, a value based in determination of a force parameter set is carried out, wherein the force parameter set is indicative of an extraction force applied to the fetal head by means of an extractor of the apparatus. In step 22, which may be carried out simultaneously to the step 21, a value-based determination of a progress parameter set is carried out, wherein the progress parameter set may be indicative of at least one of the distance between the extractor and a beacon attached stationary and separately from the extractor and a motion of the extractor relative to the beacon. In a next step 23, at least one of the progress parameter set and the force parameter set is compared with each of a plurality of data sets stored in storage means of the apparatus, wherein each data set can be indicative of a force-pattern and/or a progress-pattern recorded for previous real or virtual obstetric extraction. In a subsequent step 24, a user feedback device of the apparatus may be controlled in dependence of the comparison.

What has been stated above with regards to Fig. 1A and Fig. 1B concerning the apparatus 1 and its components, e.g., the extractor 10, the beacon 11, the sensor 12, the processor 13, and the user feedback device 14, may analogously apply to the embodiment of the method 2 schematically illustrated in Fig. 1C. Thus, to give an example, method 2 may include the step of logging, within a data logger of the apparatus, the determined force parameter set and the progress parameter set to the common time basis.

In accordance with an embodiment of the apparatus 1 schematically illustrated in Fig. 1D, the distal element 101 may be present in the form of a suction cup 101. For example, the apparatus 1 may further comprise a tube 1613 for creating a vacuum between the fetal head FH and the suction cup 101. The tube 1613 may be attached to a vacuum pump (not illustrated) of the apparatus 1. For example, the processor 13 is configured to control said vacuum pump. Further, the beacon 11 may be attached to the mons pubis 110 of the birthing mother MO. This location of the birthing mother MO can be considered to be substantial stationary with regards to the birth canal.

The apparatus 1 may further comprise a leg module 19 that may be fixed, e.g., strapped to a leg of the birthing mother MO. The leg module 19 may be mechanically and electrically coupled to the distal element 101. Further optional aspects of the leg module 19 will be explained with respect to further drawings.

In an embodiment, each of the beacon 11 and the leg module 19 are configured to transmit signals to the processor 13, such as data signals.

The display unit 142, of which an enlarged and exemplarily illustration is presented in Fig. 27, may be controlled by the processor 13. For example, the processor 13, of which some or all parts may be installed within the same housing as the display unit 142, may be configured to cause the display unit 142 to display various data, e.g., one or more of the following:
- in a section 142-1, general data such as date, time, name of the organization, name of the birthing mother MO, a registration number of the birthing mother, a current status of the processor 13;
- in a section 142-2, an overall magnitude of the applied extraction force ("traction force"), a magnitude of directional components F_{Z}, F_{X}, F_{Y} of the applied extraction force, the extraction force over time and/or the extraction force over the fetal decent (i.e., extraction force over progress);
- in a section 142-3, the number of pulls ("Pull Count"), the total time of the obstetric extraction ("VE time"), a result of said outcome prediction, e.g., the probability of an unsatisfactory outcome of the obstetric extraction, a result of said classification ("Normal VE"), a progress achieved by the recent pull, the total progress achieved, and/or the vacuum pressure.

Thus, the combination of at least parts of the processor 13 and parts of the user feedback device 14, (said combination also being referred to as "computer" in this specification), may be configured to provide one or more resources, e.g., software, display screen, user interface, e.g., a touch screen, peripheral interface, processing power, data storage, document/report generation, print interface, network connectivity and/or wireless connectivity. Illustrated in Fig. 27 is an exemplary embodiment of a computer 2701 which may be realized as, for example, a tablet, a laptop, a desktop, a system-on-chip with interface/display or other suitable arrangements. If the standard wireless connectivity resources available on the computer 2701 such as Bluetooth, Wi-Fi or the alike are insufficient, a wireless connectivity device 2702 can be employed. This device may allow connection to a computer's USB port or the alike using an appropriate connector. A circuit-board of the computer 2701 may incorporate ancillary electronics, and separate or multiplexed compliant RF, Bluetooth, Wi-Fi or similar transceivers, in order to communicate with the other components of the apparatus 1, such as with one or more of the distal element 101, the proximal element 102, the beacon 11, the leg module 19.

The computer 2701 may enable access control and may be configured to protect patient information. It may allow to secure data storage, e.g., on the data storage means 132, and may be configured to facilitate connection with a central database (not illustrated) which may be arranged remotely from the computer 2701.

In an embodiment, parts of the algorithms exemplarily described herein may be implemented on additional processor units, e.g., coupled to the wireless connectivity device 2702 so that some processing from the processor 13 can is off-loaded.

Further, computer 2701 may be configured to enable communication with other equipment that is used during the extraction, e.g., as explained above, control of the vacuum pump and display the vacuum pressure applied on the fetal head FH. In yet another instance, the computer allows entry of medical information (e.g. rate of maternal contractions, amount of cervical dilation etc.). In a further instance, the computer enables connectivity with patient records/journals for data entry or retrieval. All supplementary information (e.g. other equipment etc.) thus generated may be utilized to enhance the algorithms according to one or more embodiments.

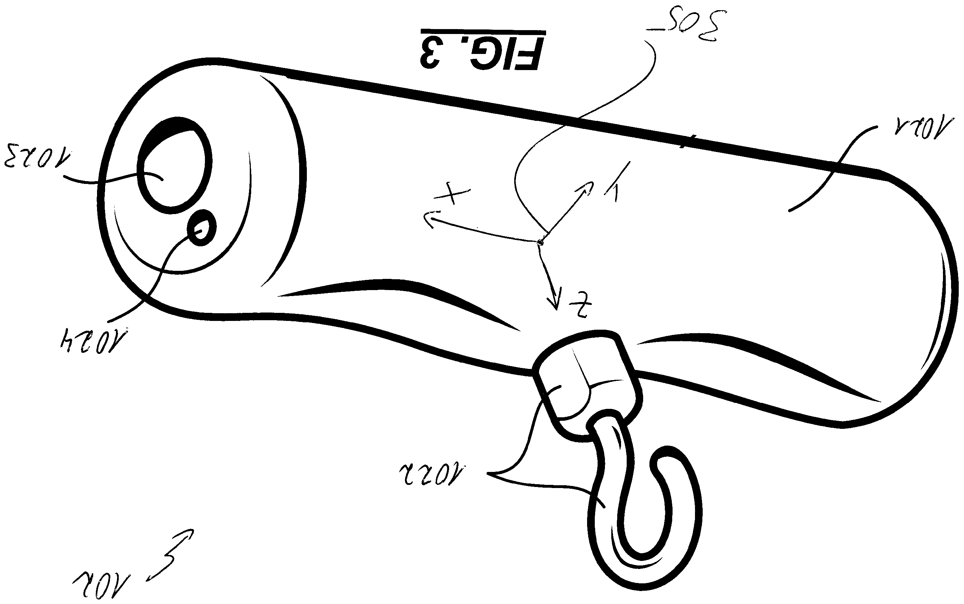

In the following, optional aspects of the proximal element 102 shall be explained with respect to Fig. 3 to Fig. 8.

In accordance with an embodiment schematically illustrated in Fig. 3, the proximal element 102 may exhibit the shape of a handle or a grip that can be gripped by hand, e.g., by a hand of a doctor (user) supervising the delivery.

For example, the proximal element 102 includes a handle 1021, wherein a housing of the handle 1021 may be made out of or comprise one or more materials, such as a plastic, a metal, or another suitable material. The handle 1021 may exhibit an ergonomic design that may allow it to be held effectively by hand while pulling.

The proximal element 102 may further include a coupling element 1022 that may be configured to be attached to connection means for connecting with the distal element 101 as, e.g., schematically illustrated in Fig. 1D.

The proximal element 102 may further include a button 1023, e.g., a power on/off button, and a display 1024 that may be configured to display a status of the proximal element 102 while in use.

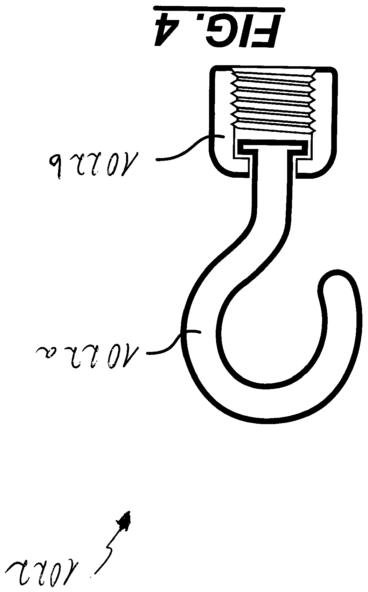

Fig. 4 schematically illustrates a section of a cross-section of the coupling element 1022 of the proximal element 102. For example, the coupling element 1022 comprises a hook 1022a that may be configured for an attachment to a chain, a string or the alike that interconnects to the distal element 101, as schematically illustrated in Fig. 1D. The hook 1022a may be attached to the handle 1021, e.g., by means of a nut 1022b, for example.

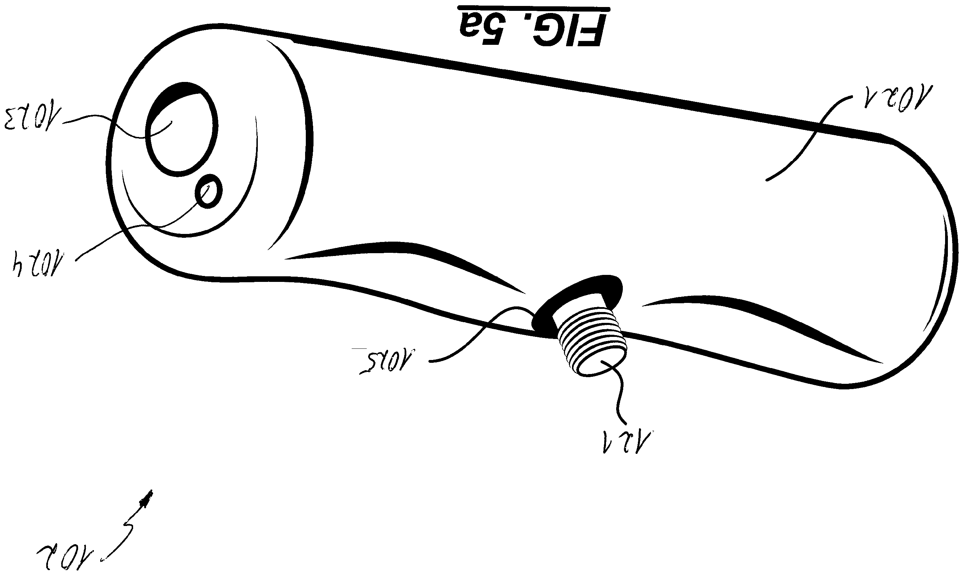

In accordance with an embodiment schematically illustrated in Fig. 5a, the force sensor 12 comprises a screw-like element 121 which is configured to be screwed into the nut 1022b of the coupling element 1022 of the proximal element 102.

At a region 1025 of the proximal element 102, where the screw-like element 121 of the force sensor 12 may protrude from, the handle 1021 may be hermetically sealed, e.g., with a material having a low shore hardness, e.g., by an O-ring, a silicon sealant or the alike.

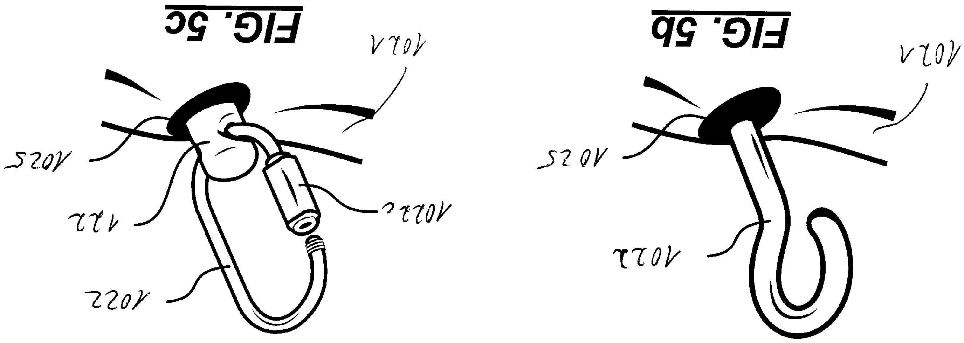

The coupling element 1022 can be realized in different ways. For example, in accordance with the embodiment schematically illustrated in Fig. 5b, hook-like means of the coupling element 1022 are permanently joined with the force sensor 12.

In another instance shown in Fig. 5c, a cylindrical part of the force sensor 122 protruding out of the handle 1021 has a recess through which the coupling element 1022, e.g., a quick-link, may pass.

After the proximal element 102 is coupled with the distal element 101, a nut 1022c of the quick-link can be secured. In further instances, a chain with a carabiner at the end or other suitable arrangement may be used as the handle-coupling.

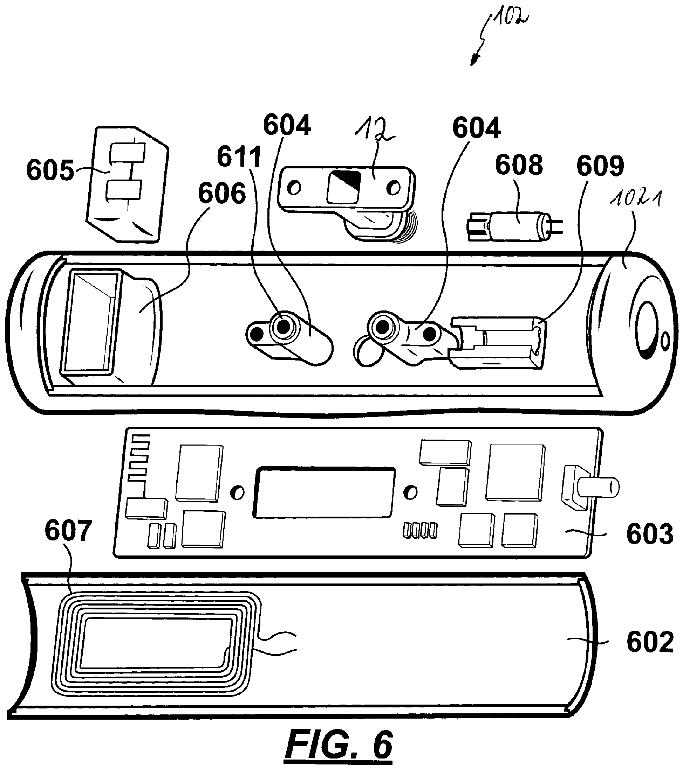

Fig. 6 schematically illustrates further optional features of the proximal element 102 which shall now be explained in more detail. As explained above, the proximal element 102 may comprise the handle 1021, wherein a housing of the handle 1021 may comprise an instrumentation of the proximal element 102. This instrumentation of the proximal element 102 may include one or more elements, e.g., the sensor 12 and/or the haptic feedback means 141.

Shown in Fig. 6 is a rearward projection of a dismantled handle 1021 of an exemplary embodiment that comprises a lid 602 which on assembly may be hermetically sealed together with the housing of the handle 1021 using, e.g., gasket, epoxy resin or the alike. A circuit-board 603 incorporating electronics of the proximal element 102 can be installed, e.g., bolted on mounting posts 604. A rechargeable battery unit 605 may be installed in a battery compartment 606. Ancillary electronics that populate the circuit-board 603 may include one or more of a button or touch switch for power on/off, status indicators, power management, power supplies and charge management. A receiver coil 607 may enable contactless charging by placing the handle on a charging dock or similar arrangements. In another instance, a connector or contacts can be employed to charge the handle.

In an embodiment, the haptic feedback unit 141 comprises a vibrator 608 that may fit into a mounting 609 within the enclosure of the handle 1021. Electronics for driving the vibrator 608 can be included on the circuit board 603. Activating the vibrator 608 vibrates the handle 1021, thus generating haptic feedback for the user. In other instances, two or more vibrating elements 608 (e.g. one vibrating element 608 alongside each mounting post 604) are deployed for a more advanced (e.g. directional) haptic feedback. The vibrator 608 may include, e.g., an eccentric rotating mass (ERM) motor and/or a linear resonant actuator (LRA).

For example, in a scenario during a obstetric extraction that need the user's attention, the processor 13 may be configured to control the vibrator 608 such that haptic feedback notifies the user to take corrective or decisive actions such that the handle is vibrated in different ways, e.g., different intensity, duration, profile etc. of vibration. In this embodiment, the user, therefore, does not have to continuously keep track of what is happening on a computer screen or a display. Such control of the haptic feedback means 141 by the processor 13 may also eliminate the need for audio or flashing visual alarms that may induce further stress in an already tense environment of the delivery room.

Further, the proximal element 102 may be attached to or comprise the force sensor 12, which can be present, e.g., in the form of a 3-axis force sensor that may be configured to measure the applied extraction force during the extraction. The force sensor 12 may be installed on the mounting posts 604.

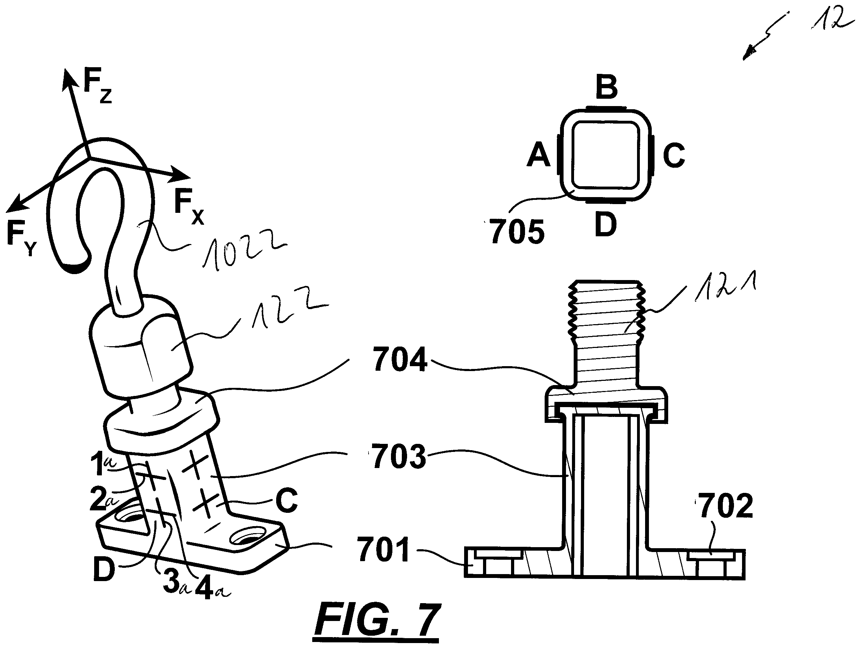

Fig. 7 schematically depicts an embodiment of the force sensor 12. The force sensor 12 can be configured to be installed, e.g., bolted on the mounting posts 604 by means of, e.g., a flange 701. For example, a boss 611 on the mounting post 604 and a matching groove 702 in the flange 701 may allow for precise positioning of the force sensor 12 within the proximal element 102.

As shown in Fig. 7, the exemplarily embodiment of the force sensor 12 may comprises a sensing beam 703 in the middle and an attachment means 704 on top. Said attachment means 704 may comprise said cylindrical element 122. A part of the attachment means 704 may protrude out of the handle 1021 and can be realized according to the design of the handle-coupling described earlier.

The force sensor 12 can be aggregated as one part and may be manufactured from the same material (e.g. plastics, stainless steel, etc.). In another instance, the flange 701 and the sensing beam 703 are manufactured from one material (e.g. stainless steel) and the attachment means 704 is manufactured from another material (e.g. plastics). Thereupon, the sensing beam 703 and the attachment means 704 are conjoined using suitable methods (e.g. molding, mechanical fixture, etc.).

According to an embodiment, the applied extraction force can be resolved into three components F_{X}, F_{Y} and F_{Z} by the force sensor 12, as illustrated in Fig. 7. For example, due to the design of the coupling element 1022, no torque could be applied. The force sensor 12 may be based on strain gauges. Further, the force sensor 12 may be configured to sense all three components F_{X}, F_{Y} and F_{Z} of the applied extraction force.

For example, said first force signal component mentioned further above may be indicative of the axial component Fz of the applied extraction force, and said second force signal component mentioned further above may be indicative of the bending component of the applied extraction force, which may include at least one of F_{X} and F_{Y}.

The sensing beam 703 may have a hollow square-shaped cross-section 705 as illustrated in Fig. 7 and its four faces are identified as A through D in Fig. 7. For example, four strain gauges may be mounted on each face with the orientation of their principle-axes represented by lines 1a through 4a. The strain gauges on each face are thus identified as, for example, on face A the four strain gauges are A1, A2, A3 and A4. The strain gauges may be connected in three separate Wheatstone bridge circuits, which may be part of the force sensor 12.

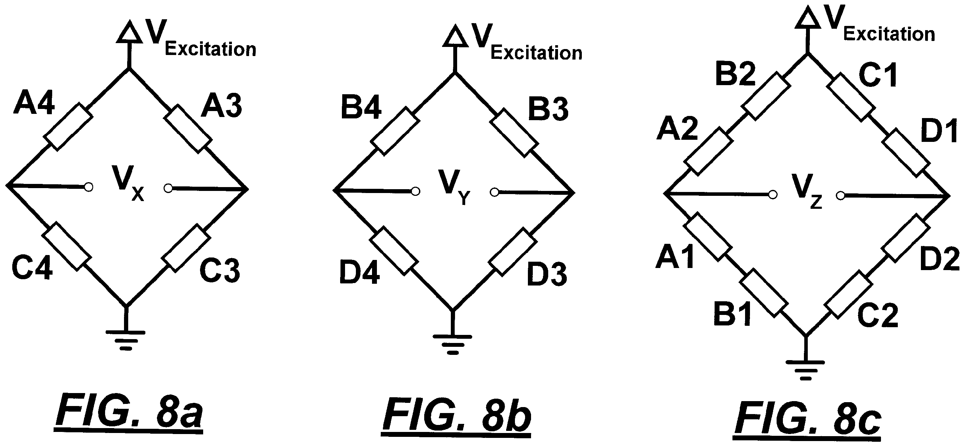

Fig. 8a, Fig. 8b and Fig. 8c depict exemplary embodiments of such Wheatstone bridge circuits that may be part of the force sensor 12 with output signals Vx, V_{Y} and V_{Z} relating to the sensing of F_{X}, F_{Y} and F_{Z} respectively. Thus, said first force signal component of the sensor signal provided by sensor 12 may comprise output signal Vx and said second signal component of the sensor signal provided by sensor 12 may comprise output signal V_{Y} and/or output signal V_{Z}.

In an embodiment, the placement and connections of strain gauges may occur such that it is ensured that the output from each Wheatstone bridge circuit is only sensitive to the related traction force component, e.g., such that V_{X} is not sensitive to F_{Y} or F_{Z}.

Other known approaches may be exploited in the design of the force sensor 12, e.g., different mechanical construction, such as use of a circular or cross-shaped sensing beam; different type, number, placement and connections of strain gauges; different force transducers etc..

The output signals V_{X}, V_{Y} and V_{Z} from the strain gauge circuits can be connected to the circuit board 603 by suitable means, e.g., flex PCB, wires etc..

In an embodiment, after optional signal-conditioning, which may include, e.g., one or more of amplification, offsetting, zero-balancing, testing etc., the output signals Vx, V_{Y} and V_{Z} are provided to the processor 13 as the force signal. The force signal may be digitized by the processor 13 and calibrated to values of force. This step may be carried out by the processor 13 during said value-based determination of the force parameter set in dependence of the force signal. The result may be a digital representation of the applied extraction force having the components F_{X}, F_{Y} and F_{Z} in suitable units, e.g., Newton.

The processor 13 may be configured to control the instrumentation of the proximal element 102, e.g., the haptic feedback means 141, and may perform other computations as well.

Thus, it shall be understood that at least parts of the processor 13 may also be implemented within the proximal element 102 of the extractor 10.

For example, the proximal element 102 comprises transceiver means that are configured for bidirectional communication with parts of the processor 13 that arranged external of the proximal element 102. E.g., the force signal is transferred in real-time, e.g. wirelessly and/or wirebound, to parts of the processor 13 arranged external of the proximal element 102, e.g., within computer 2701 mentioned above.

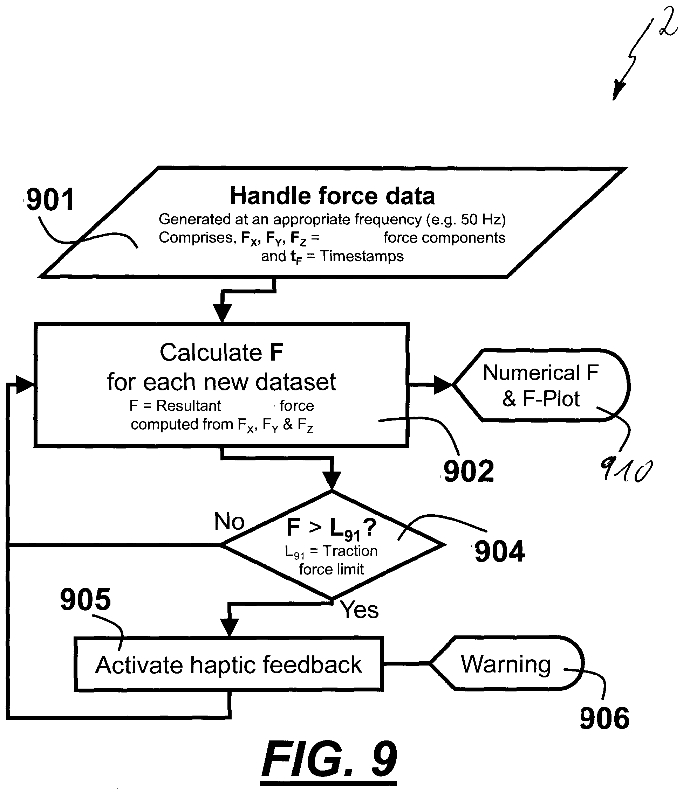

Fig. 9 schematically illustrates an optional aspect of an embodiment of the method 2 of operating the apparatus 1. For example, the method 2 is automatically carried out by the processor 13.

According to the algorithm presented in Fig. 9, in step 901, the force sensor signal generated by the force sensor 12 is received by the processor 13. The force components F_{X}, Fy and F_{Z} overtime t_{F} measured by the sensor 12 in step 901 are used to compute the applied extraction force (also referred to as "force" or "traction force" or "pull force") in step 902, i.e., to carry out said value based determination of the force parameter set.

The processor 13 may induce that the resultant traction force is displayed on the display unit 142 (cf. Fig. 1D and Fig. 27) both numerically and as a plot (Force vs time) in a section 142-2 of the display unit 142 (cf. step 910). During an extraction, if the resultant traction force is above a limit, e.g., above 350 N (cf. step 904), the processor 13 may induce that relevant haptic feedback can be activated in step 905 by means of the haptic feedback unit 141. This notifies the user that the applied extraction force should be reduced. Meanwhile, the processor 13 may also induce that a warning is displayed on the computer 2701 (e.g., display unit 412) in step 906. The limit may represent a force level at which the risk of a pop-off of the distal element 101 from the fetal head FH or other obstetric extraction anomalies is very high and can be derived using data collected from obstetric extractions in preliminary studies. All relevant data/information generated in accordance with this embodiment can be recorded in a memory and/or a database, e.g., in the storage means 132, for objective documentation.

### THE FOLLOWING IS A DETAILED DESCRIPTION OF FURTHER OPTIONAL ASPECTS OF EXEMPLARILY EMBODIMENTS.

What follows is a description of further optional aspects of exemplary embodiments, wherein this description may include illustrations of purely exemplary modes of practicing exemplary embodiments.

For example, the processor 13 may be configured to automatically carry out one or more of the algorithms presented below, wherein each of these algorithms may constitute an exemplary embodiment of the method 2.

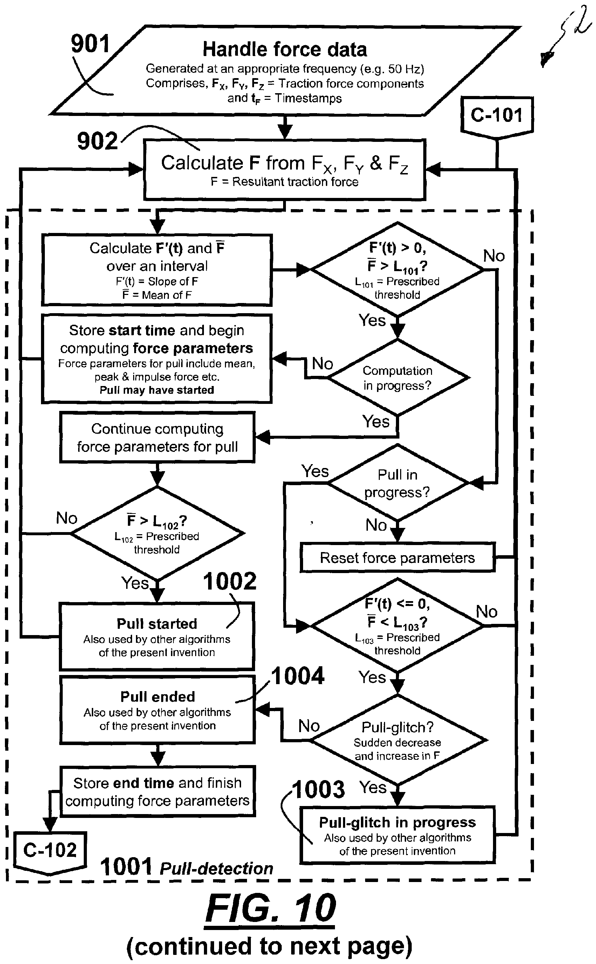

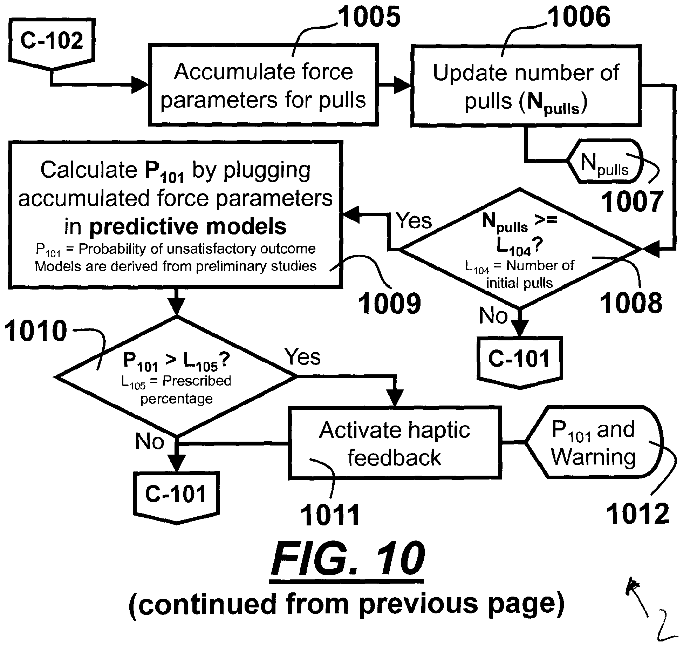

In an embodiment of the method 2, the algorithm presented in Fig. 10 is carried out, e.g., by the processor 13.

The algorithm may allow for a pull-detection and an outcome-prediction of an obstetric extraction. A pull described herein is the application of traction force during a maternal contraction. An exemplary pull-detection sequence 1001 (dashed rectangle) of the algorithm detects the start 1002 and end 1004 of a pull. This can be accomplished by comparing the slope and mean of the resultant traction force 902 (computed from handle force data 901) over an interval to a series of prescribed threshold values (derived from preliminary studies) as shown in Fig. 10.

During a pull, due to the user's technique, strength or other reasons, the traction force may drop to a very low level or zero for short intervals. Such an event is referred to as a pull-glitch. By deploying appropriate filters, the exemplary algorithm detects when a pull-glitch is in progress 1003. The pull start 1002, end 1004 and pull-glitch in progress 1003 events can be used by other algorithms. Enhancements in the pull-detection sequence 1001 may be made for improved results. From the resultant traction force 902, said value based determination of the force parameter set can be computed for each pull.

The force parameter set may include one or more force parameters, e.g., peak and mean force for a pull; force impulse (integral of force over time) for a pull; and difference between force impulse of the recent pull and the pull before it. Some force parameters can be accumulated and used in further computations 1005. Other parameters that are also computed include, but are not limited to, start time, end time and duration of a pull. A pull count or total number of pull 1006 can also be maintained and displayed on the computer 2701 in step 1007.

An obstetric extraction can have an unsatisfactory outcome described earlier. In an embodiment, using data collected from obstetric extractions with known outcomes in preliminary studies, models that predict the outcome of an obstetric extraction are deduced by employing statistical, pattern-matching or similar techniques. For example, the aforesaid may be carried out by means of said comparison of the determined force parameter set with the data sets that may be stored in storage means 132.

As depicted in Fig. 10, after a prescribed number of initial pulls (e.g. after first three pulls) 1008, predictive models derived for the present algorithm use the accumulated force parameters to calculate the probability of unsatisfactory outcome (as a percentage) 1009. E.g., this value demonstrates the chances of an ongoing obstetric extraction to have an unsatisfactory outcome. If the probability of unsatisfactory outcome is above a prescribed percentage (e.g. 80%) 1010, relevant haptic feedback is activated 1011 to notify the user, and a warning with the probability value is displayed on the computer in step 1012. The prescribed number of initial pulls and the prescribed percentage specified above are also deduced from preliminary studies. During an obstetric extraction, a haptic feedback generated on the basis of the present algorithm, enables the user to further evaluate the ongoing situation and make an overall assessment. As a consequence, the user may decide to end the obstetric extraction immediately (i.e. a safe early-exit) and opt a C-section instead. An early-exit saves crucial time and resources, and the mother and baby from unnecessary trauma. All relevant data/information generated in this embodiment of the handle is recorded to memory/database for objective documentation.

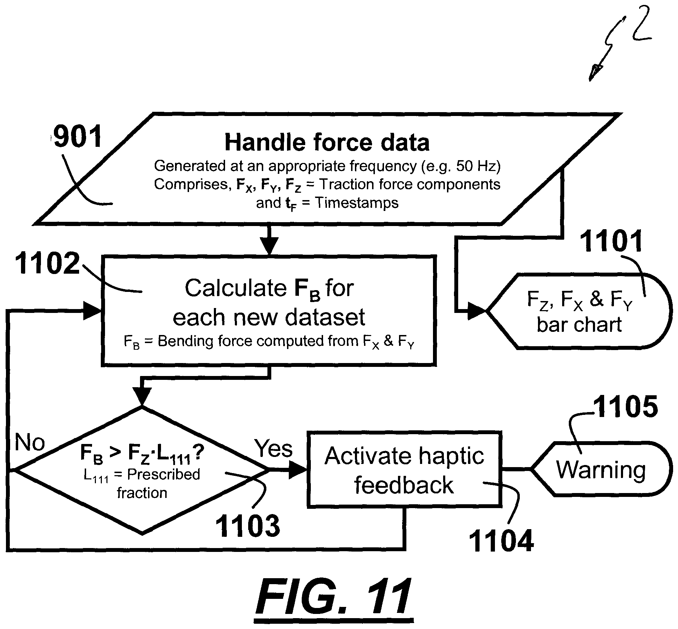

In a further embodiment, the algorithm presented in Fig. 11 allows quality-control of an obstetric extraction. The handle force data 0901 comprises the traction force components F_{X}, F_{Y} and F_{Z} which are displayed on the computer using suitable representation such as a bar chart 1101. F_{X} and F_{Y} are the bending components whereas F_{Z} is the axial component of the traction force. The resultant bending force (FB) is computed from F_{X} and F_{Y} 1102. F_{Z} is also referred to as the axial force. If the handle is used correctly the axial force should be predominant and the bending force should be minimal. High bending force depicts incorrect action of pulling and an increased risk of pop-off. As shown in Fig. 11, if the bending force is more than a prescribed fraction (e.g. 30%; deduced from preliminary studies) of the axial force 1103, relevant haptic feedback is activated 1104 to notify the user, and a warning is displayed on the computer 1105. Consequently, the user can consult the bar chart 1101 and improve the action of pulling thus maintaining or improving the quality of an obstetric extraction. All relevant data/information generated in this embodiment of the handle is recorded to memory/database for objective documentation.

In a further embodiment, transceiver means, such as a distance sensor, are included in the extractor 10, which may allow for progress-monitoring of an obstetric extraction.

The distance sensor may be based on, for example, ultrasound, optical markers and camera, infrared, inductive or other known technologies. The choice of technology is relatively trivial compared to the required functionality (i.e. distance measurement from the beacon 11). The distance sensor can be based on Ultra-Wide-Band (UWB) radar technology.

Suitable UWB distance sensors include, but are not limited to: a transmitter and receiver pair from Messtechnik, Ortung und Datenverarbeitung GmbH (MEODAT), Germany; and time-of-flight or time-difference-of-flight implementations. The MEODAT implementation described herein uses active-approach, where the transmitter and receiver cooperate with each other and should thus be synchronized using methods described later. The receiver or acquisition domain of the UWB distance sensor can be populated on the circuit-board 603 in the enclosure of the handle 1021 (Fig. 6) and comprises, but is not limited to, an antenna, a SiGe receiver IC (e.g. with track and hold) and a signal processing unit. In a manner to be described, the supplementary transmitter of the UWB distance sensor is incorporated in an embodiment of the beacon 11.

The beacon 11 can be affixed to the mother's mons pubis and by virtue of its location, and may provide a reference point on the mother's body with respect to which the descent of the fetal head within the birth canal can be measured.

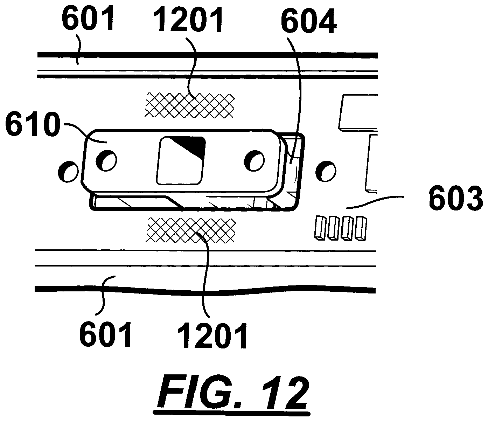

Fig. 12 partially shows an embodiment according to which the circuit-board 603 and the force sensor 12 are assembled within the same enclosure. The opposite side of the cross-hatched region 1201 in the middle of the circuit-board 603 is a possible location to place the antenna of the UWB receiver. In another instance, for improved distance measurements, two UWB receivers may be deployed, with their antennas located at the edges of the circuit-board 603.

Using the progress signal received from the transmitter, the processing unit of the UWB receiver computes the distance between the handle 1021 and the beacon 11 in suitable units (e.g. centimeters) which constitutes handle distance data, which may be part of said progress parameter set.

For example, while a pull is being exerted, for the reason that the coupling between the handle 1021 and the cup 101 is fully extended, the handle 1021 retains a fixed distance from the fetal head FH. Therefore, an increase in distance between the handle 1021 and the beacon 11 means an increase in distance between the fetal head FH and the beacon 11, which essentially represents the descent of fetal head FH.

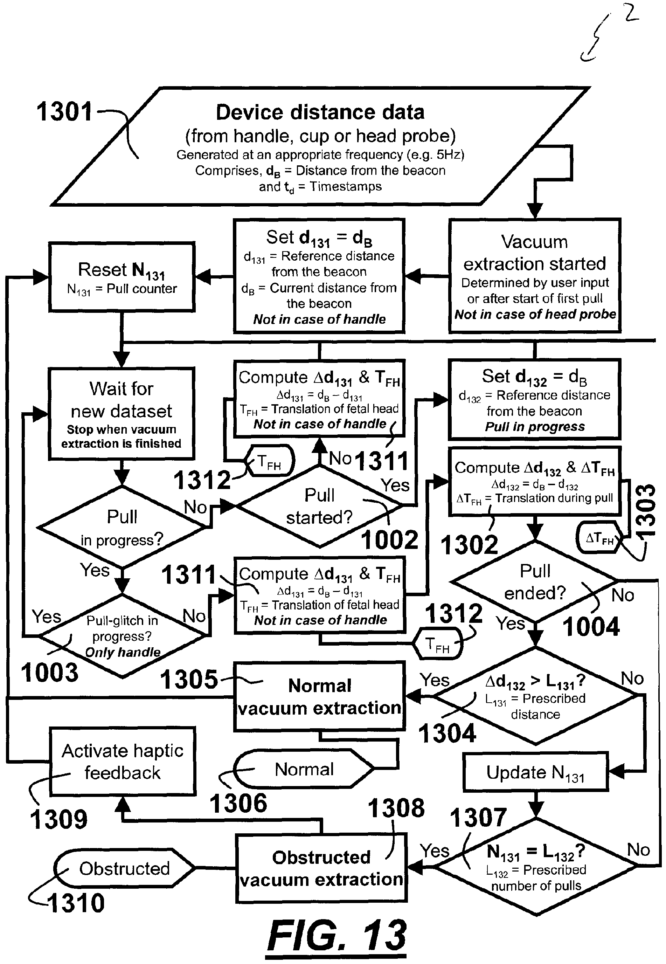

The exemplary algorithm presented in Fig. 13 exploits this property of distance measurements from the handle. Using such device distance data (handle distance data in this case) 1301 during an obstetric extraction, the algorithm computes the pull-related change in distance between the handle and the beacon 1302 during a pull (i.e. after the start 1002 and up to the end 1004 of a pull). The distance between the handle and the fetal head may not remain fixed during pull-glitches, and this is also accounted for 1003.

Since the birth canal is curved in shape, the pull-related change in distance is also converted into pull-related translation of fetal head 1302 and is displayed on the computer 1303. After completion of a pull 1004, if the distance between the handle and the beacon increases above a prescribed distance (e.g. 0.5 cm) 1304, this implies that the fetal head is following; such an obstetric extraction is classified as normal 1305 and this status is displayed on the computer 1306. On the other hand, if the distance between the handle and the beacon does not increase above the prescribed distance 1304 for a prescribed number of pulls (e.g. three pulls) 1307, this implies that the fetal head is not following; such an obstetric extraction is classified as obstructed 1308 and relevant haptic feedback is activated 1309 to notify the user, and a warning with this status is displayed on the computer 1310. The user can thus make an overall assessment and may decide to end the obstetric extraction immediately (i.e. an early-exit). The prescribed distance and the prescribed number of pulls specified above are deduced from preliminary studies. All relevant data/information in this embodiment of the handle is transferred to the computer in real-time through the transceiver 603 on the circuit-board 603 (Fig. 6) and is also recorded to memory/database for objective documentation.

In a further embodiment, a motion sensor in the handle may allow for progress-monitoring of an obstetric extraction. The motion sensor may be based on, for example, magnetic, optical markers and camera or other known technologies. The choice of technology is relatively trivial compared to the required functionality (i.e. measurement of change in position and orientation of the handle). The motion sensor exemplified in the this embodiment is based on MEMS inertial sensor technology. The constituents of the inertial motion sensor are mounted on the circuit-board 603 (Fig. 6) and suitable components include, but are not limited to: a 9-axis sensor IC 603 (MotionTracking device with gyroscope, accelerometer and compass) from InvenSense Inc., USA under the model names MPU-9255, MPU-9250 and MPU-9150; and a motion processor 603 such as an ARM Cortex-M4. The 9-axis sensor IC 603 has a known position within the handle with respect to the handle frame 305 shown in Fig. 3. The motion processor 603 uses data from the 9-axis sensor IC 603 and sensor fusion software (e.g. InvenSense Embedded MotionDriver) to generate the handle motion data. The handle motion data comprises, but is not limited to, gyroscope data (3-axis angular velocities in degrees per second), accelerometer data (3-axis accelerations in gees), compass data (3-axis magnetic field in micro tesla), handle quaternions (sensor fused rotational angles), linear acceleration and gravity vector.

Other sensor technologies may generate different motion data. For example, the 3D guidance system from Ascension Technology Corp., USA generates position data of 6-DOF sensors with respect to an electromagnetic transmitter. For the exemplary inertial motion sensor in the handle, a supplementary motion sensor may be incorporated in the beacon which generates the beacon motion data. The beacon motion data is identical to the handle motion data and comprises gyroscope data, accelerometer data, etc. By virtue of anatomy, the movement of the fetal head (not due to labor or pulling), the mons pubis and the birth canal, due to mother's external body movements are equivalent. The beacon which is affixed to the mother's mons pubis, thus indirectly provides data related to the motion of the fetal head due to mother's body movements. While a pull is being exerted, for the reason that the coupling between the handle and the cup is fully extended, the handle retains a fixed distance from the fetal head. Therefore, the movement of the handle in the direction of pull represents the descent of fetal head within the birth canal.

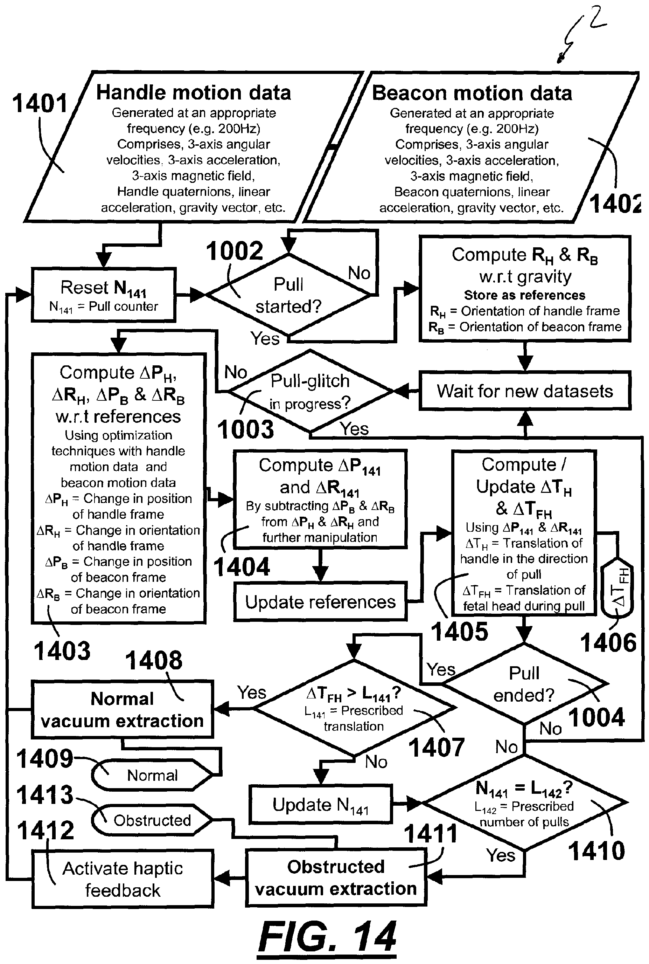

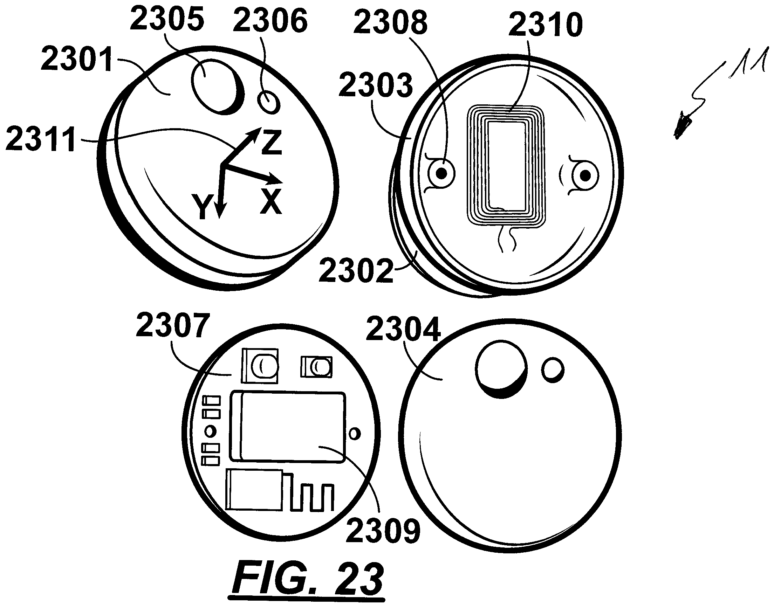

Said property is exploited by the algorithm presented in Fig. 14. After start of a pull 1002 and up to its end 1004, by applying system modelling, estimation, drift-reduction and other optimization techniques for motion capture, on the handle motion data 1401 and the beacon motion data 1402; changes in the position and orientation of the handle frame (305 in Fig. 3) and the beacon frame (2311 in Fig. 23) are computed 1403 after accounting for pull-glitches 1003. The change in position and orientation of the handle frame 305 is related to the movement of the fetal head due to pulling and mother's external body movements. The change in position and orientation of the beacon frame 2311 represents mother's external body movements. By subtracting the change in position and orientation of the beacon frame from the change in position and orientation of the handle frame and further manipulations, the effects of mother's external body movements are omitted.

The resultant change in position and orientation of the handle frame 1404 is thus closely linked with the descent of the fetal head due to pulling. The resultant change in position and orientation of the handle frame is used to compute the pull-related translation of handle along the negative Z-axis of the handle frame (i.e. the direction of pull) in suitable units (e.g. centimeters) 1405. This pull-related translation of handle essentially represents the pull-related translation of fetal head 1405 and is displayed on the computer 1406. After completion of a pull 1004, if the pull-related translation of fetal head is above a prescribed translation (e.g. 0.5 cm) 1407, this implies that the fetal head is following; such an obstetric extraction is classified as normal 1408 and this status is displayed on the computer 1409. On the other hand, if the pull-related translation of fetal head stays below the prescribed translation 1407 for a prescribed number of pulls (e.g. three pulls) 1410, this implies that the fetal head is not following; such an obstetric extraction is classified as obstructed 1411 and relevant haptic feedback is activated 1412 to notify the user, and a warning with this status is displayed on the computer 1413. The user can thus make an overall assessment and may decide to end the obstetric extraction immediately (i.e. an early-exit). The prescribed translation and the prescribed number of pulls specified above are deduced from preliminary studies. All relevant data/information in this embodiment of the handle is transferred to the computer in real-time through the transceiver 603 on the circuit-board 603 (Fig. 6) and is also recorded to memory/database for objective documentation.

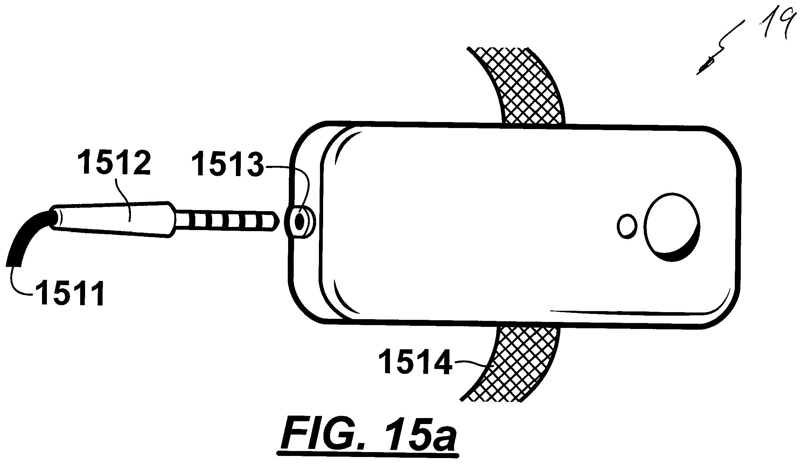

The leg module 19 may facilitates instrumentation. The affiliate devices of the leg module 19 may include, but are not limited to, the cup, a head probe 15 or a combination thereof. The exemplary leg module 19 shown in Fig. 15a has a housing with a rectangular or other suitable shape that may be fastened to the mother's leg using a strap 1514, an adhesive pad or other suitable means.

The materials used to manufacture the leg module 19 may include, plastics, metals, other suitable materials or a combination thereof. If the leg module 19 is deployed, only some constituents of the optional instrumentation (e.g. transducers, sensors etc.) are directly attached to the affiliate device.

A cable 1511 originating from the affiliate device with a connector 1512 at its end is used to connect the device with the leg module 19 through the connector 1513. The design of the connectors (1512 and 1513) ensures that their function is not compromised due to exposure to body fluids and provisions are made that allow them to be disinfected after use.

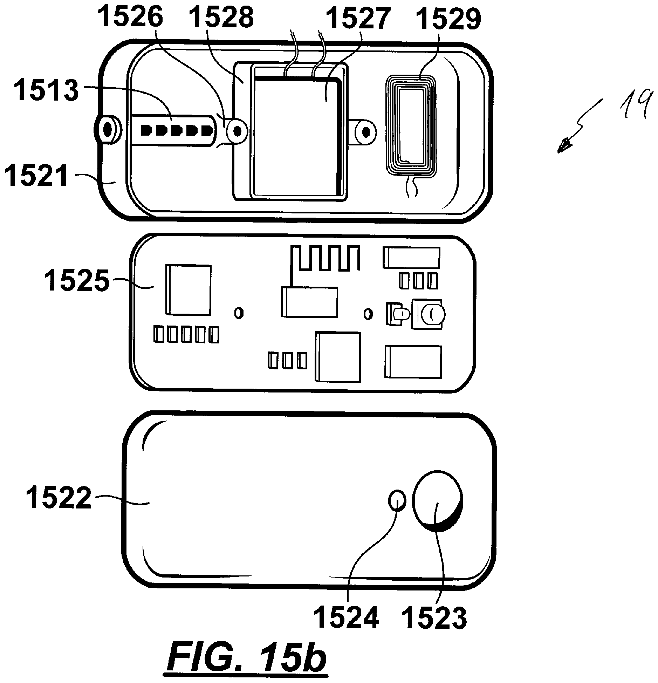

In order not to obtrusive, cable management solutions are employed and the number of cables is minimized. As shown in Fig. 15b, the leg module 19 comprises an enclosure 1521 and a cover 1522 which on assembly are hermetically sealed together using gasket, epoxy resin or the alike. The cover 1522 has means that allow power on/off 1523 and display the status of the device 1524. A circuit-board 1525 (may be more than one) incorporating the electronics of the leg module 19 is mounted on the bosses 1526 in the enclosure 1521. A rechargeable battery unit 1527 is installed in the battery compartment 1528. The ancillary electronics for the device that populate the circuit-board 1525 includes, but is not limited to, a button or touch switch for power on/off 1525, status indicators 1525, power management 1525, power supplies 1525 and charge management 1525. Also available on the circuit-board 1525 is, e.g., a transceiver 1525 that complies with one or more embodiments. A receiver coil 1529 or the alike enables contactless charging by placing the leg module 19 on a charging dock or similar arrangements. In another instance, a connector or contacts are used to charge the leg module 19. Further electronics in the leg module 19 depends on the sensors used in the affiliate devices.

The instrumented cup can be passed through the mother's cervix and attached to the fetal head by means of vacuum pressure. Traction force is then applied on the cup during a maternal contraction using the handle, to pull the fetal head out of the mother.

Some embodiments of the cup provide identical functionalities to the handle.

Having these functions only in the handle can circumvent additional instrumentation in the cup. However, having them in the cup instead may have, for example, more accurate results.

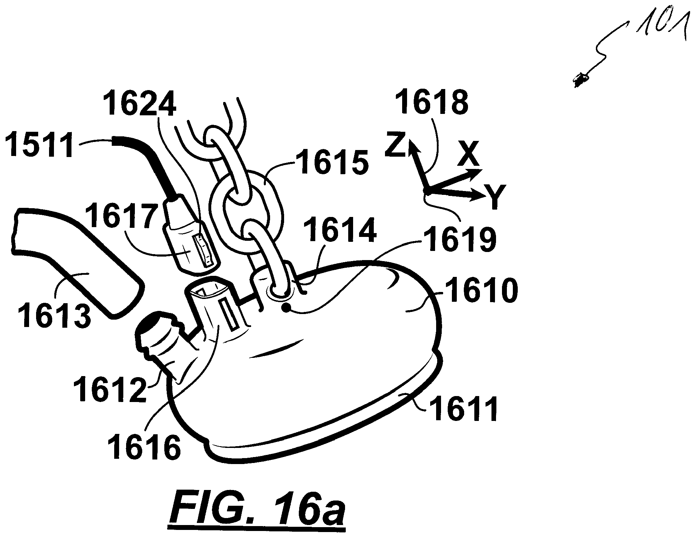

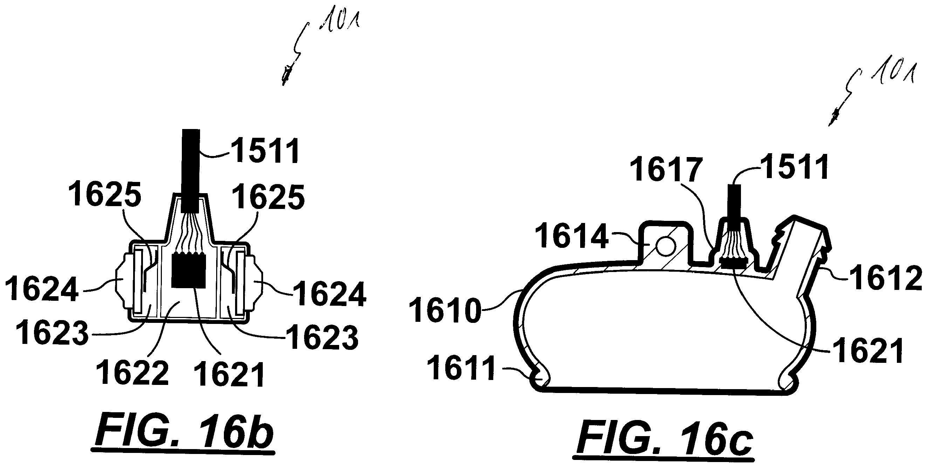

An embodiment of the distal element 101, which may be present in the form of said cup, is exemplified in Fig. 16. The materials used to manufacture the cup and its constituents may include, plastics, composites, metals, other suitable materials or a combination thereof. The embodiment illustrated in Fig. 16a, comprises a mushroom-shaped member 1610 with an annular-lip 1611 that provides air-tight seal when held against the fetal head under vacuum pressure. A duct 1612 situated on the edge of the member 1610 allows connection to a vacuum pump (e.g. with a silicon tube 1613) that evacuates the interior of the member 1610 thus attaching the cup firmly to the fetal head. A cylindrical protrusion or knob 1614 joined at the center of the member 1610 allows the cup-coupling 1615 to be appended to the cup. In other instances, the knob 1614 may be replaced by a D-link, a shackle or other suitable arrangements. The cup-coupling 1615 allows the cup to be attached to the handle-coupling described previously. As shown in Fig. 16a, the cup-coupling 1615 is realized as a chain made up of a suitable number of rings, with the first ring passing through a hole in knob 1614. The rings of the chain 1615 can be slightly twisted to keep the chain flat when extended. In other instances, a string or other suitable arrangements may be used as cup-coupling. Situated at an appropriate location on the member 1610 is a slotted-socket 1616 that enables a sensor module 1617 to be affixed to the cup. Some elements of the cup instrumentation (e.g. a sensor or transducer) are incorporated in the sensor module 1617.

Fig. 16b depicts the internal arrangement of the sensor module. The relevant sensor or transducer 1621 (to be described) with a cable 1511 originating from it, is potted within the central cavity 1622. The cavity 1622 is hermetically sealed using epoxy resin or the alike. Cavities 1623 on either side of the central cavity 1622 house ear-like bodies 1624 pushed outwardly by spring elements 1625. This arrangement allows the sensor module 1617 to be snapped into the slotted-socket 1616 shown in Fig. 16a.

By pressing together both ear-like bodies 1624, the sensor module 1617 may be released from the slotted-socket 1616. In other instances, a screw-on, a clip-on, a sliding rail with locking means or other suitable arrangements may be used to secure the sensor module on the cup.

Fig. 16c depicts the cross-section of yet another instance, where a hermetically sealed sensor module 1617 is permanently attached to the cup member 1610 using suitable means (e.g., by an adhesive or epoxy, by molding or over-molding etc.). The cable 1511 originating from the sensor module 1617 passes out of the mother's body to be connected externally to the leg module 19 (described earlier) that incorporates the remainder of the cup instrumentation. In a further instance, an enlarged sensor module encapsulates the entire instrumentation of the cup along with the sensor or transducer; thus eliminating the leg module 19 and omitting the need for the cable 1511. The embodiments described hereinafter may employ a leg module 19 but their constituents can be incorporated as a stand-alone device.

In a further embodiment, a distance sensor in the cup allows progress-monitoring of an obstetric extraction. The distance sensor may be based on, for example, ultrasound, optical markers and camera, infrared, inductive or other known technologies. The choice of technology is relatively trivial compared to the required functionality (i.e. distance measurement from the beacon). The distance sensor exemplified in this embodiment is based on Ultra-Wide-Band (UWB) radar technology. Suitable UWB distance sensors include, but are not limited to: a transmitter and receiver pair from Messtechnik, Ortung und Datenverarbeitung GmbH (MEODAT), Germany; and time-of-flight or time-difference-of-flight implementations. The MEODAT implementation described herein uses active-approach, where the transmitter and receiver cooperate with each other and should thus be synchronized using methods described later. The receiver or acquisition domain of the UWB distance sensor comprises, but is not limited to: an antenna with a SiGe receiver IC (e.g. with track and hold) 1621 incorporated in the sensor module 1617 (Fig. 16); and a signal processing unit 1525 populated on the circuit-board 1525 in the leg module 19 (Fig. 15). In another instance, only the antenna 1621 is incorporated in the sensor module 1617 (Fig. 16) and the SiGe receiver IC with the signal processing unit 1525 are populated on the circuit-board 1525 in the leg module 19 (Fig. 15). The cable 1511 connects the sensor module 1617 to the leg module 19. In yet another instance, two or more UWB receivers may be employed for improved distance measurements. In a manner to be described, the supplementary transmitter of the UWB distance sensor may be incorporated in an embodiment of the beacon. The beacon is affixed to the mother's mons pubis and by virtue of its location, provides a reference point on the mother's body with respect to which the descent of the fetal head within the birth canal can be measured. Using the signal received from the transmitter, the processing unit of the UWB receiver computes the distance between the cup and the beacon in suitable units (e.g. centimeters) which constitutes the cup distance data. Since the cup remains firmly attached to the fetal head during an obstetric extraction, the cup distance data can be used without interruptions (instead of only during pulls) and has better attributes (e.g. span, damping, stability etc.) as compared to its counterpart from the handle. Furthermore, an increase in distance between the cup and the beacon means an increase in distance between the fetal head and the beacon, which essentially represents the descent of fetal head. The algorithm presented in Fig. 13 exploits this property of distance measurements from the cup. Using device distance data (cup distance data in this case) 1301, the algorithm computes the overall change in distance between the cup and the beacon 1311 after the start of an obstetric extraction. Due to the curved shape of the birth canal, the overall change in distance is converted into overall translation of fetal head 1311 and is displayed on the computer 1312. After the start 1002 and up to the end 1004 of a pull, the pull-related change in distance between the cup and the beacon is computed 1302. The corresponding pull-related translation of fetal head is also computed 1302 and displayed on the computer 1303. After completion of a pull 1004, if the pull-related change in distance between the cup and the beacon is above a prescribed distance (e.g. 0.5 cm) 1304, this implies that the fetal head is following; such an obstetric extraction is classified as normal 1305 and this status is displayed on the computer 1306. On the other hand, if the pull-related change in distance between the cup and the beacon stays below the prescribed distance 1304 for a prescribed number of pulls (e.g. three pulls) 1307, this implies that the fetal head is not following; such an obstetric extraction is classified as obstructed 1308 and relevant haptic feedback is activated 1309 to notify the user, and a warning with this status is displayed on the computer 1310. The user can thus make an overall assessment and may decide to end the obstetric extraction immediately (i.e. an early-exit). The prescribed distance and the prescribed number of pulls specified above are deduced from preliminary studies. All relevant data/information in this embodiment of the cup is transferred to the computer in real-time through the transceiver 1525 on the circuit-board 1525 in the leg module 19 (Fig. 15) and is also recorded to memory/database for objective documentation.

In a further embodiment, a motion sensor in the cup allows progress-monitoring of an obstetric extraction. The motion sensor may be based on, for example, magnetic, optical markers and camera or other known technologies. The choice of technology is relatively trivial compared to the required functionality (i.e. measurement of change in position and orientation of the cup). The motion sensor exemplified in this embodiment is based on MEMS inertial sensor technology. Suitable constituents of the inertial motion sensor include, but are not limited to: a 9-axis sensor IC 1621 (MotionTracking device with gyroscope, accelerometer and compass) from InvenSense Inc., USA under the model names MPU-9255, MPU-9250 and MPU-9150, incorporated in the sensor module 1617 (Fig. 16); and a motion processor 1525 such as an ARM Cortex-M4 populated on the circuit-board 1525 in the leg module 19 (Fig. 15). The cable 1511 originating from the sensor module 1617 connects it to the leg module 19. When the sensor module 1617 is affixed on the cup (e.g. in the slotted-socket 1616), the 9-axis sensor IC 1621 has a known position with respect to the cup frame 1618 located at the point 1619 as depicted in Fig. 16. The motion processor 1525 uses data from the 9-axis sensor IC 1525 and sensor fusion software (e.g. InvenSense Embedded MotionDriver) to generate the cup motion data. The cup motion data comprises, but is not limited to, gyroscope data (3-axis angular velocities in degrees per second), accelerometer data (3-axis accelerations in gees), compass data (3-axis magnetic field in micro tesla), cup quaternions (sensor fused rotational angles), linear acceleration and gravity vector. For the motion sensor in the cup, a supplementary motion sensor is incorporated in an embodiment of the beacon which generates the beacon motion data. The beacon motion data is identical to the cup motion data and comprises gyroscope data, accelerometer data, etc. By virtue of anatomy, the movement of the fetal head (not due to labor or pulling), the mons pubis and the birth canal, due to mother's external body movements are equivalent. The beacon which is affixed to the mother's mons pubis, thus indirectly provides data related to the motion of the fetal head due to mother's body movements. Since the cup remains firmly attached to the fetal head during an obstetric extraction, the cup motion data can be used without interruptions (instead of only during pulls) and has better attributes (e.g. span, damping, stability etc.) as compared to its counterpart from the handle. Furthermore, the movement of the cup in the direction outwards of the mother's body represents the descent of fetal head in the birth canal.

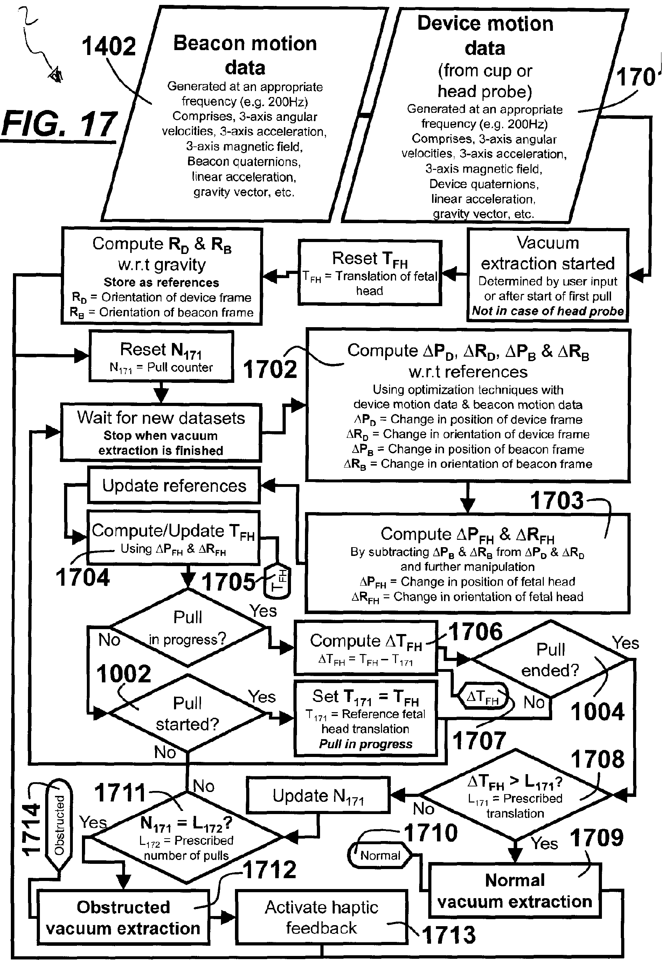

This property is exploited by the algorithm presented in Fig. 17. After an obstetric extraction starts, by applying system modelling, estimation, drift-reduction and other optimization techniques for motion capture, on the device motion data (cup motion data in this case) 1701 and the beacon motion data 1402; changes in the position and orientation of the device frame (cup frame 1618 shown in Fig. 16 in this case) and the beacon frame (2311 in Fig. 23) are computed 1702. The change in position and orientation of the device (cup) frame 1618 represents the movement of the fetal head due to labor, pulling and mother's external body movements. The change in position and orientation of the beacon frame 2311 represents mother's external body movements. By subtracting the change in position and orientation of the beacon frame from the change in position and orientation of the device (cup) frame and further manipulations, the effects of mother's external body movements are omitted. As a result, the change in position and orientation of the fetal head only due to labor or pulling is obtained 1703. From this, the overall translation of fetal head due to labor or pulling along the positive Z-axis of the device (cup) frame (i.e. the direction outwards of the mother's body) is computed 1704, and is also displayed on the computer 1705. After the start 1002 and up to the end 1004 of a pull, the pull-related translation of fetal head is also computed 1706 and displayed on the computer 1707. After completion of a pull 1004, if the pull-related translation of fetal head is above a prescribed translation (e.g. 0.5 cm) 1708, this implies that the fetal head is following; such an obstetric extraction is classified as normal 1709 and this status is displayed on the computer 1710. On the other hand, if the pull-related translation of fetal head stays below the prescribed translation 1708 for a prescribed number of pulls (e.g. three pulls) 1711, this implies that the fetal head is not following; such an obstetric extraction is classified as obstructed 1712 and relevant haptic feedback is activated 1713 to notify the user, and a warning with this status is displayed on the computer 1714. The user can thus make an overall assessment and may decide to end the obstetric extraction immediately (i.e. an early-exit). The prescribed translation and the prescribed number of pulls specified above are deduced from preliminary studies. All relevant data/information in this embodiment of the cup is transferred to the computer in real-time through the transceiver 1525 on the circuit-board 1525 in the leg module 19 (Fig. 15) and is also recorded to memory/database for objective documentation.

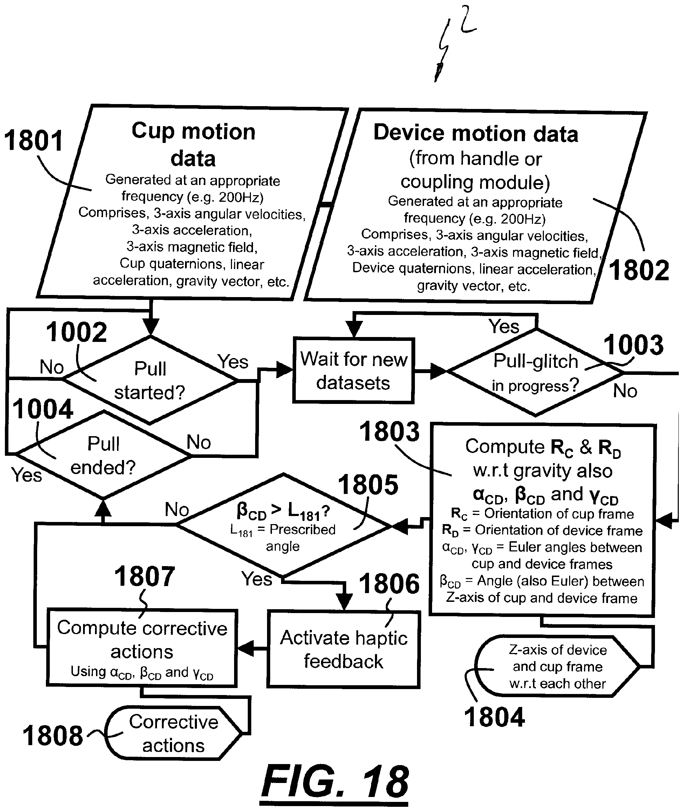

In a yet further embodiment, the algorithm presented in Fig. 18 allows quality-control of an obstetric extraction. In order to reduce the risk of pop-offs and efficient transfer of applied force to the cup during an obstetric extraction, the Z-axes of the cup frame 1618 (Fig. 16) and the handle frame 305 (Fig. 3) should be aligned (i.e. angle between the axes should be close to zero degrees); so that the applied traction force is centered and perpendicular to the annular-lip 1611 (Fig. 16) of the cup. Between the start 1002 and end 1004 of a pull, the algorithm uses the cup motion data 1801 and the device motion data 1802 (handle motion data in this case), to compute the absolute orientation of the cup and handle frames with respect to gravity 1803. Using matrix algebra or similar techniques the relative angles between the axes of the cup and the handle frames are thus computed 1803. These angles represent the orientation of the cup and the handle with respect to each other. The orientation of the Z-axes of the cup and the handle frames with respect to each other is displayed on the computer using appropriate representation (e.g. graphical, text etc.) 1804. If the angle between the Z-axes of the cup and the handle frames is above a prescribed angle (e.g. 30 degrees; deduced from preliminary studies) 1805, meaning that the Z-axes are not aligned, relevant haptic feedback is activated 1806 to notify the user. Based on the orientation of cup and the handle with respect to each other, corrective actions (e.g. move handle upwards, downwards etc.) are computed 1807 and displayed on the computer using appropriate representation (e.g. graphical, text, etc.) 1808. In another instance, more advanced haptic feedback (e.g. guiding motion) within the handle suggests which corrective action should to be taken. In some case, depending on the sensor technology adopted for the motions sensors in the cup and the handle, an initial alignment or homing sequence may be required before the presented algorithm starts execution. All relevant data/information in this embodiment is transferred to the computer in real-time and is also recorded to memory/database for objective documentation.

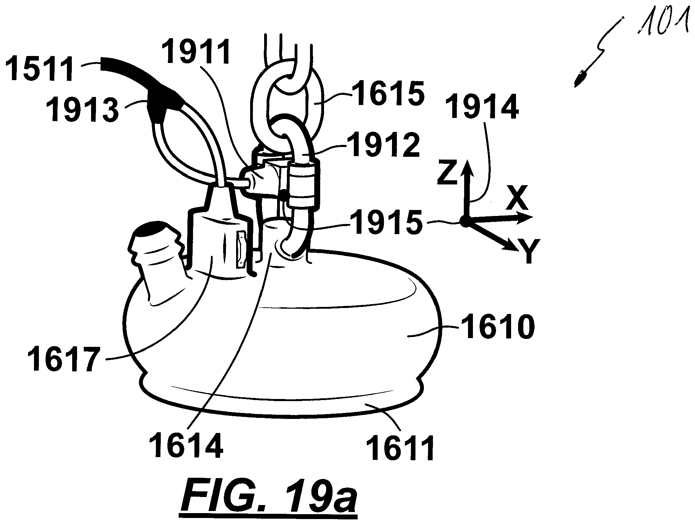

In an embodiment, a motion sensor in the cup-coupling allows quality-control of an obstetric extraction. The cup-coupling motion sensor exemplified herein is identical to the inertial motion sensor explained above with respect to other embodiments. Fig. 19a depicts an arrangement where a cup-coupling module 1911 is attached to a modified first ring 1912 of the cup-coupling (chain) 1615. The ring 1912 passes through a hole in the knob 1614 on the cup member 1610. The sensor module 1617 housing the 9-axis sensor IC 1621 (Fig. 16) may be affixed on the cup. Cables originating from the cup-coupling module 1911 and the sensor module 1617 merge at the junction 1913, thus transforming into a single cable 1511.

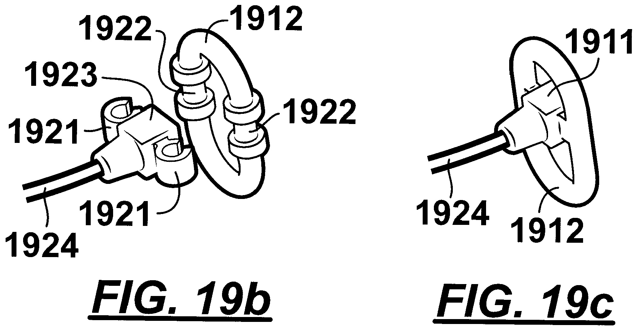

As depicted in Fig. 19b, the cup-coupling module comprises an arrangement with two c-shaped clips 1921 that snap on to the sheaves 1922 on the ring 1912. The hermetically sealed capsule 1923 houses the 9-axis sensor IC (e.g. from InvenSense Inc., USA) of the cup-coupling motion sensor with the cable 1924 originating from it. On assembly the cup-coupling module is firmly secured to the cup-coupling 1615. In other instances, a screw-on, a click-on or other suitable arrangements may be used to secure the cup-coupling module to the cup-coupling 1615.

Yet another instance is depicted in Fig. 19c, where a hermetically sealed cup-coupling module 1911 is permanently attached to the ring 1912 using suitable means (e.g., by an adhesive or epoxy, by molding or over-molding etc.). When the cup-coupling module 1911 is affixed to the cup-coupling 1615, the 9-axis sensor IC within the cup-coupling module 1911 has a known position with respect to the cup-coupling frame 1914 located at the point 1915 as depicted in Fig. 19a. The cable 1511 passes out of the mother's body to be connected externally to a leg module 19 that incorporates the remainder of the instrumentation. A motion processor 1525 populated on the circuit-board 1525 of the leg module 19 (Fig. 15), uses data from the 9-axis sensor IC in the cup-coupling module 1911, to generate the coupling motion data. This data can be identical to the cup motion data also generated, and described above. In order to reduce the risk of pop-offs and efficient transfer of applied force to the cup during an obstetric extraction, the Z-axes of the cup frame 1618 (Fig. 16) and the cup-coupling frame 1914 (Fig. 19) should be aligned (i.e. angle between the axes should be close to zero degrees); so that the applied traction force is centered and perpendicular to the annular-lip 1611 (Fig. 16) of the cup. Also included in this embodiment of the cup, is the algorithm presented in Fig. 18. Between the start 1002 and end 1004 of a pull, the algorithm uses the cup motion data 1801 and the device motion data 1802 (coupling motion data in this case), to compute the absolute orientation of the cup and cup-coupling frames with respect to gravity 1803. Using matrix algebra or similar techniques the relative angles between the axes of the cup and the cup-coupling frames are also computed 1803. These angles represent the orientation of the cup and the coupling with respect to each other. The orientation of the Z-axes of the cup and the cup-coupling frames with respect to each other is displayed on the computer using appropriate representation (e.g. graphical, text etc.) 1804. If the angle between the Z-axes of the cup and the cup-coupling frames is above a prescribed angle (e.g. 30 degrees; deduced from preliminary studies) 1805, meaning that the Z-axes are not aligned, relevant haptic feedback is activated 1806 to notify the user. Based on the orientation of cup and the cup-coupling with respect to each other, corrective actions (e.g. move handle upwards, downwards etc.) are computed 1807 and displayed on the computer using appropriate representation (e.g. graphical, text, etc.) 1808. In another instance, more advanced haptic feedback (e.g. guiding motion) within the handle suggests which corrective action should to be taken. In some case, depending on the sensor technology adopted for the motions sensors in the cup and the cup-coupling, an initial alignment or homing sequence may be required before the above algorithm starts execution. All relevant data/information in this embodiment of the cup is transferred to the computer in real-time through the transceiver 1525 on the circuit-board 1525 in the leg module 19 (Fig. 15) and is also recorded to memory/database for objective documentation.

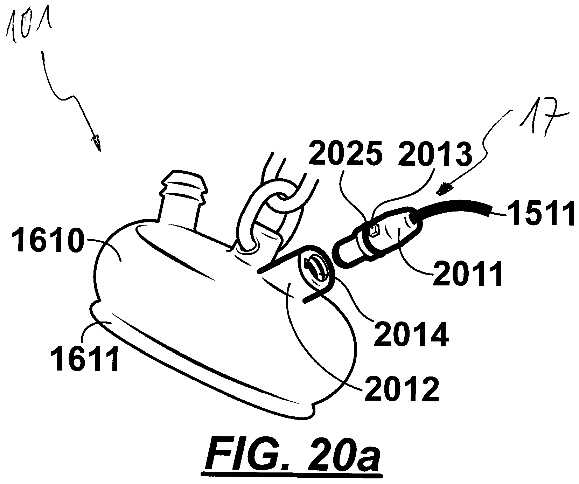

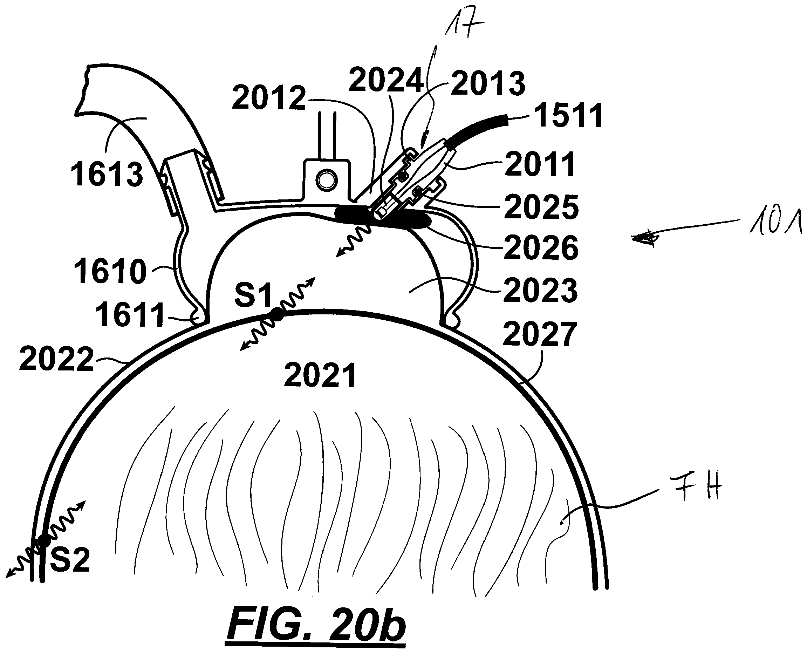

In a yet further embodiment, a skull sensor 17 in the cup instrumentation allows detection of excessive elongation of fetal head. The arrangement exemplified in Fig. 20a comprises a skull module 2011 that is inserted into the slanted socket 2012 on the cup member 1610. The skull module 2011 is inserted into the socket 2012 with the key 2013 aligned with the keyhole 2014. Once fully inserted, rotating the skull module 2011 secures it firmly in place. In other instances, a screw-on, a click-on or other suitable arrangements may be used to secure the skull module on the cup. In yet another instance, a hermetically sealed skull module is permanently attached to the cup member 1610 using suitable means (e.g., by an adhesive or epoxy, by molding or over-molding etc.). In a further instance, sensor module of the distance sensor or the motion sensor in an embodiment of the cup may also be housed in the skull module. The constituents of the skull sensor 17 include, but are not limited to, an ultrasound transducer in the skull module and a processor (e.g. an ARM Cortex-M4) 1525 along with intermediary electronics (power converters, switches, amplifiers, etc.) 1525 populated on the circuit-board 1525 in the leg module 19 (Fig. 15). The processor 1525 controls the ultrasound transducer through the intermediary electronics 1525. Suitable ultrasound transducers include, but are not limited to, high frequency transducers (typically 1MHz and up) from Ceram AB, Sweden; Sonometrics Corporation, Canada; SECO Sensor Consult GmbH, Germany; and Olympus Corporation, Japan. Illustrated in Fig. 20b, is the cross-section of the cup held under vacuum pressure against the fetal head 2021 in the birth canal. The vacuum pressure causes the fetal scalp 2022 to be sucked into the cup, forming a bulge 2023. The cross-section of a fully inserted skull module 2011 is also shown, with the ultrasound transducer 2024 of the skull sensor 17 encapsulated in the front end of the skull module 2011. The cable 1511 originating from the ultrasound transducer 2024 connects it to the associated instrumentation in the leg module 19. An O-ring 2025 or similar means ensure that there is no loss of vacuum pressure from the arrangement. A hole in the member 1610 within the slanted socket 2012 allows the ultrasound transducer 2024 to reach the interior of the cup member 1610 at an angle. To ensure no air gaps between the ultrasound transducer 2024 and the fetal scalp 2022, a gel pad (ultrasound conducting medium e.g. hydrogel) 2026 is appended to the interior surface of the cup member 1610, where it comes in contact with the ultrasound transducer 2024. Due to vacuum pressure, the bulge 2023 presses against the gel pad thus enabling seamless travel of ultrasound signals from the ultrasound transducer 2024 to the fetal head 2021 and back. The processor 1525 (Fig. 15) of the skull sensor 17 employs the ultrasound transducer 2024 both as a transmitter and a receiver.

As depicted in Fig. 20b, the generated ultrasound pulses travel through tissue and are reflected from the points S1 and S2 on the fetal skull 2027. Since the speed of sound in body tissue is known, the time between the generation of ultrasound pulses and their reflections from points S1 and S2 on the fetal skull 2027 are used to compute the distances dS1 and dS2 between the ultrasound transducer 2024 and the points S1 and S2 respectively. These distance measurements generated by the processor 1525 of the skull sensor 17 constitute the skull data. During delivery, if the head gets wedged, pulling only elongates it, thus giving a false sense that the head is following.

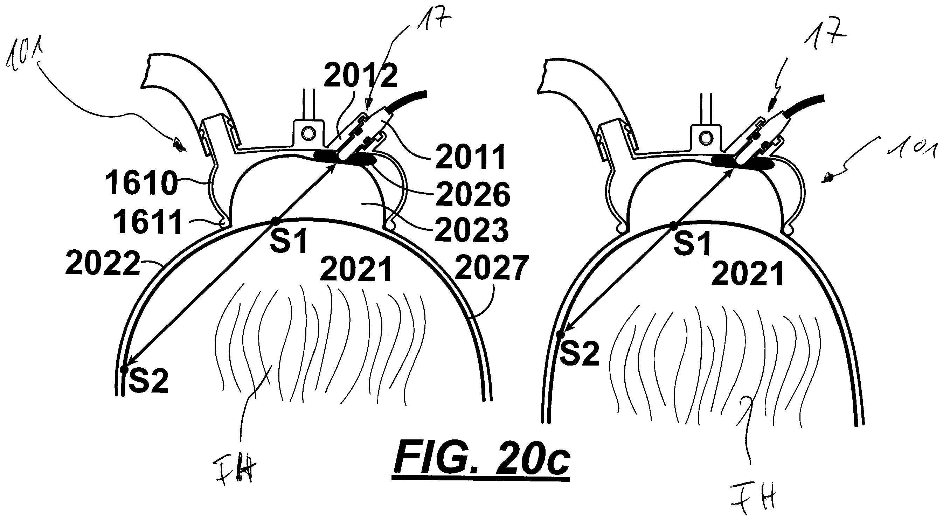

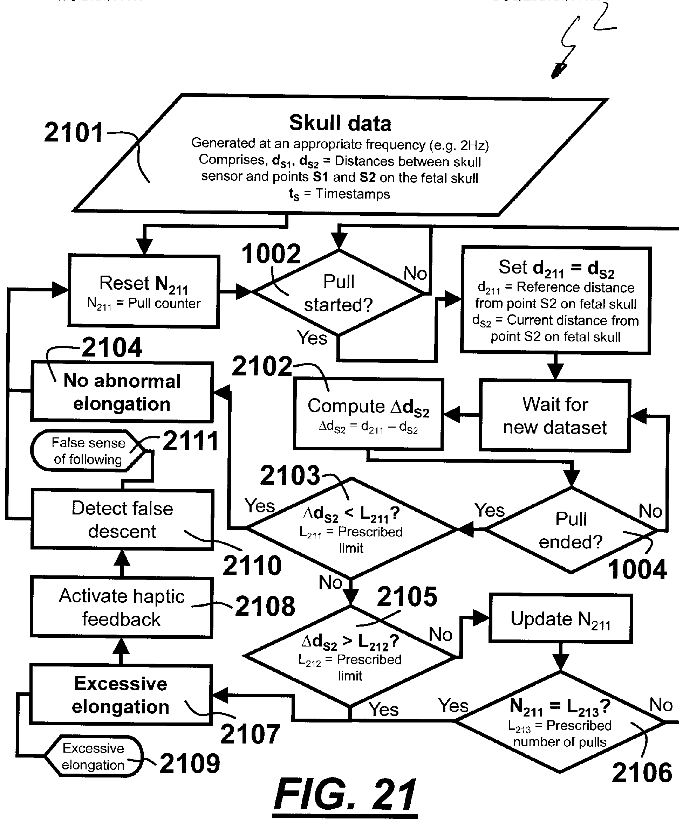

On the left side of the Fig. 20c, a normal fetal head (FH) 2021 together with the cup and the skull module 2011 is shown, whereas on the right side an elongated fetal head 2021 is depicted. It is evident from Fig. 20c that the elongation of the fetal head causes the distance dS2 to decrease. The algorithm presented in Fig. 21 exploits this property of the distance dS2 in the skull data 2101. After the start 1002 and up to the end 1004 of a pull, the change in distance dS2 is computed 2102. After the completion of a pull 1004, a change in distance dS2 below a prescribed lower limit 2103, implies that there is no abnormal elongation in the fetal head 2104. On the other hand, if the change in distance dS2 is above a prescribed upper limit 2105, or is between the prescribed lower limit and the prescribed upper limit for a prescribed number of pulls 2106, this implies that the fetal head has excessive elongation 2107 and may be wedged. Relevant haptic feedback is thus activated 2108 to notify the user, and a warning with this status is displayed on the computer 2109. Furthermore, parameters including, but not limited to, for example, the ratio between the change in distance dS2 and the pull-related translation of fetal head, are used to detect if there is false descent of the fetal head 2110. If a false descent is detected, relevant warning is displayed on the computer 2111. After receiving the above feedback, the user can make an overall assessment and may decide to end the obstetric extraction immediately (i.e. an early-exit). All prescribed values detailed herein are deduced from preliminary studies. All relevant data/information in this embodiment of the cup is transferred to the computer in real-time through the transceiver 1525 on the circuit-board 1525 in the leg module 19 (Fig. 15) and is also recorded to memory/database for objective documentation. Further embodiments of the skull sensor 17 include, but are not limited to: use of other characteristics of the distance measurements from the fetal skull; use of two or more ultrasound transducer at suitable locations on the cup member 1610 (Fig. 20); use of ultrasound array transducers such as annular arrays, sector scan probes etc.; and use of other suitable technologies such as magnetic, Ultra-Wide-Band radar, etc.

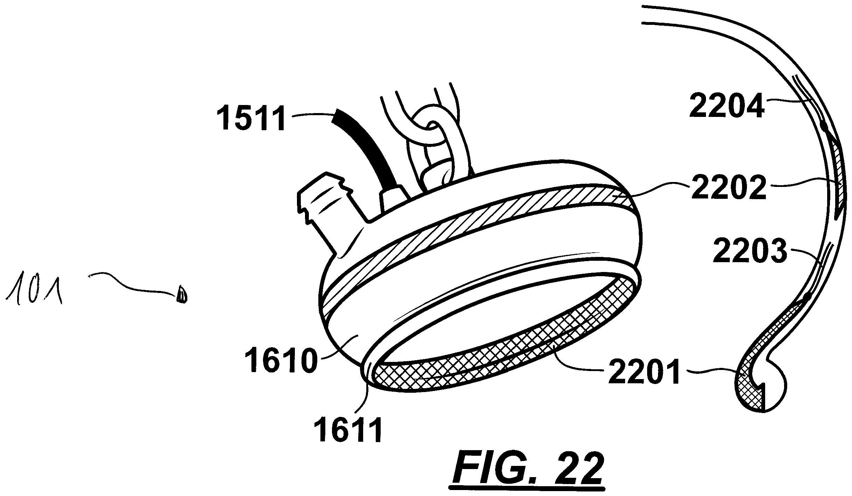

In a yet further embodiment, electrodes in the cup allow the measurement of fetal electrocardiogram (ECG) signal. The mushroom-shaped cup member 1610 depicted in Fig. 22 is manufactured from an electrically and magnetically non-conductive material (e.g. suitable plastics or the alike). Two electrodes manufactured from suitable metals (e.g. non-magnetic stainless steel) are embedded on the surface of the cup member 1610. Fig. 22 also shows a partial cross-section of the member 1610. The so-called positive electrode (crosshatched region) 2201 that lines the inner surface of the annular-lip 1611 and the member 1610, provides contact with the fetal body (head). The negative electrode (hatched region) 2202 that lines the outer bulge of the member 1610, provides contact with the amniotic fluid. Both electrodes establish the bipolar ECG lead configuration. In other instances, the said electrodes may not cover the entire circumference of the cup or may be split into several segments. When the cup is held against the fetal head under vacuum pressure, the inner positive electrode 2201 remains insulated from the outer negative electrode 2202. Using suitable methods (e.g. soldering, crimping, etc.), two separate and electrically insulated wires or leads 2203 and 2204 are connected to the electrodes 2201 and 2202 respectively. These leads are routed on or through the member 1610 to merge into the cable 1511 from the remaining instrumentation of the cup describe previously (e.g. sensor module 1617 in Fig. 16). As the cable 1511 passes out of the mother's body, the leads 2203 and 2204 split up from it at a reasonable length and are then connected to suitable fetal monitoring equipment using appropriate connectors. Using the bipolar leads of this embodiment, the said monitoring equipment measures the fetal ECG signal and thereupon the fetal heart rate and other parameters pertaining to fetal heartbeats and well-being.

In an embodiment, the beacon 11 works in synergy with its affiliate devices that may include, e.g., the proximal element 102, e.g., the handle 1021, the distal element 101, e.g., the cup, the head probe 15 or a combination thereof. The beacon 11 may provide a reference framework for the mother's birth canal. It can be configured to be affixed to a point or location on the mother's body that due to human anatomy does not change position and orientation with respect to the birth canal. As the fetal head FH descends within the birth canal, measurements are performed relative to the said point or location. This can be a part of the value based determination of the progress parameter set to be carried out by the processor 13.

The mons pubis 110 (Fig. 1D) is one such location. Other such locations on the mother's body include, but are not limited to, the left and right anterior superior iliac spine. Described hereinafter, are exemplary embodiments of the beacon 101.

The exemplary arrangement shown in Fig. 23 comprises a housing 2301 with a disk-like or other suitable shape that is affixed firmly to the mother's mons pubis 110 using an adhesive pad 2302 or similar means. Surgical tape may be used to further secure the housing 2301. The materials used to manufacture the beacon may include, plastics, metals, other suitable materials or a combination thereof.

The beacon 11 comprises an enclosure 2303 and a cover 2304 which on assembly are hermetically sealed together using gasket, epoxy resin or the alike. The cover 2304 has means that allow power on/off 2305 and display the status of the device 2306. A circuit-board 2307 (may be more than one) incorporating the beacon electronics is mounted on the bosses 2308 in the enclosure 2303. The ancillary electronics for the device that populate the circuit-board 2307 includes, but is not limited to, a rechargeable battery unit 2309, a button or touch switch for power on/off 2307, status indicators 2307, power management 2307, power supplies 2307 and charge management 2307. A receiver coil 2310 or the alike enables contactless charging by placing the beacon on a charging dock or similar arrangements. In another instance, a connector or contacts are used to charge the beacon. Further electronics in the beacon depends on the sensors in the affiliate devices.

A further embodiment of the beacon supplements the distance sensor in the affiliate device. The instrumentation of the beacon may vary depending on the choice of distance sensing technology i.e. ultrasound, optical markers and camera etc. For Ultra-Wide-Band radar technology formerly exemplified for the affiliate devices, the transmitter or stimulation domain of the UWB distance sensor is incorporated in the beacon. Since active approach is used in the distance sensor, the transmitter in the beacon and the receiver in the affiliate device cooperate to perform distance measurements. The constituents of the transmitter are mounted on the circuit-board 2307 (Fig. 23) and suitable components include, but are not limited to: a SiGe transmitter IC from MEODAT, Germany that transmits a signal (e.g. maximum-length-binary-sequence) through an antenna; and a processor such as an ARM Cortex-M4 for synchronizing the transmitter with the receiver by software. In another instance, synchronization is achieved by deploying proximity sensors (e.g. from Omron Corporation, Japan) in the beacon and the affiliate device and holding both devices in close proximity. In yet another instance, synchronization is achieved by connecting or touching contacts made available on the beacon and the affiliate device. Other suitable methods for synchronizing the transmitter and receiver may be employed. Consequently, the beacon allows the affiliate device to measure distance from its reference location on the mother's mons pubis and generate the resulting device distance data 1301 (Fig. 13). The distance measurements may be improved further by utilizing triangulation techniques and different combinations of transmitters and receivers (e.g. using more than one beacon and/or receivers in the affiliate device).

In yet a further embodiment, the beacon comprises a motion sensor that supplements the motion sensor in the affiliate device. This embodiment of the beacon allows removal of the effects of the mother's body movements in the computations related to the descent of the fetal head within the birth canal. The instrumentation of the beacon may vary depending on the choice of motion sensing technology i.e. magnetic, optical markers and camera etc. For MEMS inertial sensor technology formerly exemplified for the affiliate devices, identical components are incorporated in motion sensor of the beacon. As shown in Fig. 23, the constituents of the motion sensor are mounted on the circuit-board 2307 and suitable components include, but are not limited to: a 9-axis sensor IC 2307 and the related software from InvenSense Inc., USA; and a motion processor 2307 such as an ARM Cortex-M4 for implementation of the software. The 9-axis sensor IC 2307 has a known position within the beacon with respect to the beacon frame 2311. Consequently, the motion processor 2307 generates the beacon motion data which is identical to the data generated by inertial motion sensors described previously. Relevant data is transferred to the computer in real-time through a transceiver 2307 on the circuit-board 2307 and is also recorded to memory/database. The beacon motion data 1402 is used by algorithms depicted in Fig. 14 and Fig. 17 for progress-monitoring of obstetric extractions.

An independent system for progress-monitoring of vaginal deliveries before an obstetric extraction is opted may be realized with an arrangement consisting of the head probe 15, the beacon 11 and the computer 13. Some embodiments of the head probe 15 are described as follows.

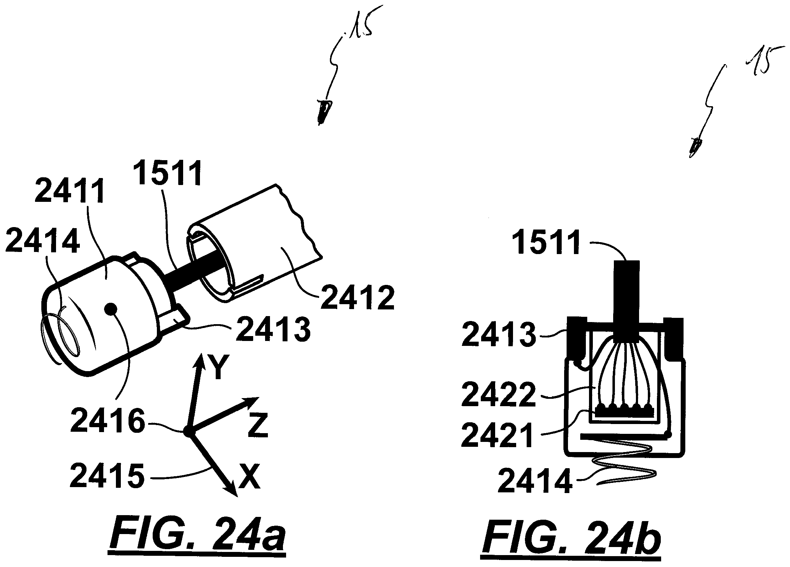

In an embodiment of the head probe 15 is exemplified in Fig. 24a. The materials used to manufacture the head probe 15 may comprise, plastics, metals, other suitable materials or a combination thereof. As shown in Fig. 24a, the head probe 15 comprises a sensor housing 2411 whose design is identical to the conventional scalp electrode invented in the 1960s. The sensor housing 2411 is attached directly to the fetal head by passing it through the mother's vagina and cervix. In order to affix the sensor housing 2411 to the head of the fetus in labor, it is first held against the head by using two fingers and a tube for guidance. Then by rotating the slotted drive tube 2412 that fits on the wings 2413 of the sensor housing 2411, the pointed and sharp spiral attachment 2414 is screwed into the epidermis of the fetal head. The driving tube 2412 is rotated until the sensor housing 2411 is firmly secured on the fetal head. In other instances, vacuum pressure, adhesives or other suitable means may be used to secure the sensor housing on the fetal head.

Shown in Fig. 24b is a cross-section of the sensor housing 2411. The relevant sensor or transducer 2421 to be described with a cable 1511 originating from it is potted within the cavity 2422. The cavity 2422 is hermetically sealed using epoxy resin or the alike. After attachment, only the sensor housing 2411 and a small length of the cable 1511 remain within the mother's body. The cable 1511 passes out of the mother's body to be connected externally to a leg module 19 that incorporates the remainder of the instrumentation of the head probe 15. In another instance, an enlarged housing encapsulates the entire instrumentation of the head probe 15 along with the sensor or transducer; thus eliminating the leg module 19 and omitting the need for the cable 1511. The embodiments described hereinafter employ a leg module 19 but their constituents can be incorporated as a stand-alone device.

A further embodiment of the head probe 15 comprises a distance sensor which is a replica of the distance sensor of an embodiment of the cup. Wherein, the sensor 2421 residing in the sensor housing 2411 in Fig. 24a comprises elements of the receiver of the UWB radar based distance sensor and is connected to the leg module 19 via the cable 1511, e.g., in an identical manner to an embodiment of the cup described earlier. Thereupon, e.g., in similar fashion to an embodiment of the cup described earlier, device distance data 1301 with respect to the beacon may be generated and the algorithm presented in Fig. 13 is used to compute the translation of fetal head 1311 during labor, which is also displayed on the computer 1312. In this embodiment, the computed translation of fetal head 1311 and the time after the head probe 15 starts measurements is used to determine and display the rate of translation of the fetal head; which is expressed, for example, in centimeters per hour. If the rate of translation is below a prescribed level the labor is assessed to have a slow progress. All relevant information/data is recorded to memory/database.

A further embodiment of the head probe 15 comprises a motion sensor which can be configured as described above. Wherein, the sensor 2421 residing in the sensor housing 2411 in Fig. 24 comprises a 9-axis sensor IC of the MEMS inertial motion sensor and is connected to the leg module 19 via the cable 1511 in an identical manner as described above. The 9-axis sensor IC 2421 has a known position within the sensor housing 2411 with respect to the head probe 15 frame 2415 located at the point 2416 as depicted in Fig. 24a. Thereupon, in a similar fashion as described above, device motion data 1701 is generated and the algorithm presented in Fig. 17 is used to compute the translation of fetal head 1704 during labor which is also displayed on the computer 1705. This embodiment uses the computed translation of fetal head 1704 and the time after the head probe 15 starts measurements to determine and display the rate of translation of the fetal head; which is expressed, for example, in centimeters per hour. If the rate of translation is below a prescribed level the labor is assessed to have a slow progress. All relevant information/data is recorded to memory/database.

A further embodiment of the head probe 15 allows the measurement of fetal ECG signal in addition to progress-monitoring. Similar to conventional scalp electrodes, the spiral attachment 2414 and the wings 2413 illustrated in Fig. 24a are both realized in suitable metals (e.g. non-magnetic stainless steel) and are electrically connected within the sensor housing 2411 to two separate leads or wires that merge into the cable 1511. The spiral attachment 2414 is attributed as the so-called positive electrode and provides contact with the fetal body (head). The wings 2413 are attributed as the negative electrode and provide contact with the amniotic fluid. Both electrodes establish the bipolar ECG lead configuration. The design and method of placement of the sensor housing 2411 ensures that the positive and negative electrode remain insulated from each other. As the cable 1511 passes out of the mother's body, the leads connected to the positive and negative electrode split up from it at a reasonable length, and are then connected to suitable fetal monitoring equipment using appropriate connectors. Using the bipolar leads in this embodiment, the said monitoring equipment measures the fetal ECG signal and thereupon the fetal heart rate and other parameters pertaining to fetal heartbeats and well-being.

Described hereinafter are algorithms constituting exemplary embodiments of the method 2 and that may provide schemes to attain some functionalities in some embodiments.

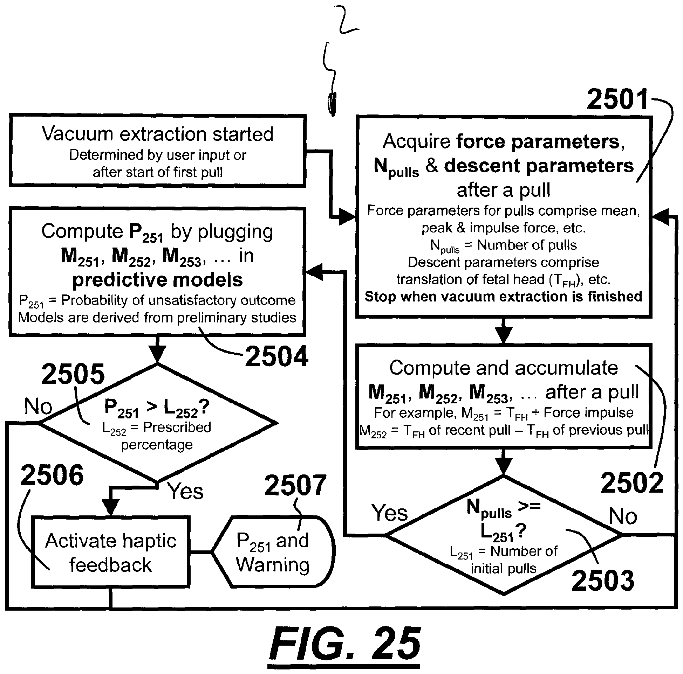

The algorithm presented in Fig. 25 allows outcome-prediction of an obstetric extraction. After a pull is completed, computed parameters from relevant embodiments of the handle or cup in use are acquired 2501. These include, force parameters comprising force impulse, peak force, mean force etc. 1005 (Fig. 10); number of pulls 1006; and descent parameters comprising translation of fetal head (e.g. 1302 or 1311 in Fig. 13), characteristics of translation of fetal head during a pull etc. Using data collected from obstetric extractions in preliminary studies, models that predict the outcome of an obstetric extraction are deduced by employing statistical, pattern-matching or similar techniques. Relevant parameters for these models are computed and accumulated 2502. These parameters include, but are not limited to, for example, the ratio between the translation of fetal head and the force impulse for a pull etc. After a prescribed number of initial pulls (e.g. after first three pulls) 2503, the predictive models derived for the present algorithm are used to calculate the probability of unsatisfactory outcome 2504. If this probability is above a prescribed limit (e.g. 80%) 2505, relevant haptic feedback is activated 2506 to notify the user and a warning with the probability value is displayed on the computer 2507. The user can thus make an overall assessment and may decide to end the obstetric extraction immediately (i.e. an early-exit). All prescribed values detailed herein are also deduced from preliminary studies. All relevant information/data is recorded to memory/database.

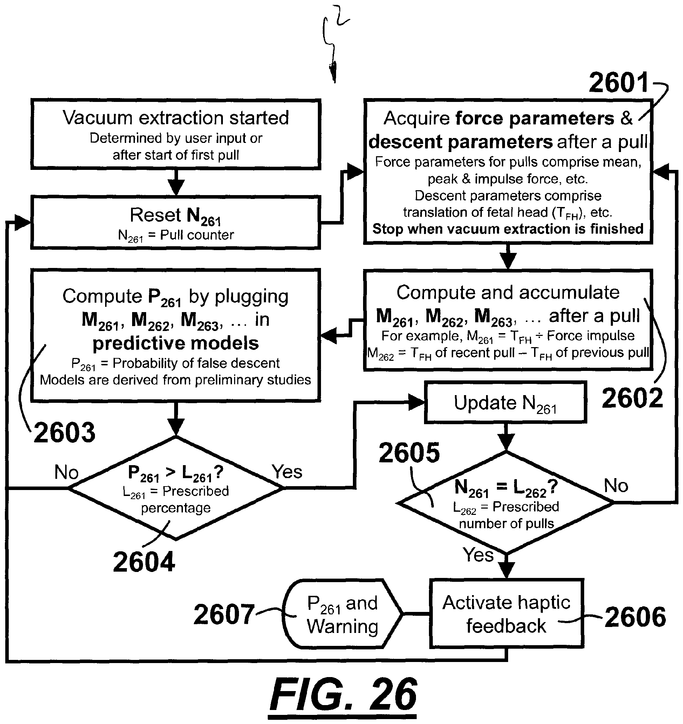

The algorithm presented in Fig. 26 allows the prediction of false descent for an obstetric extraction. After a pull is completed, computed parameters from relevant embodiments of the handle or cup in use are acquired 2601. These include, force parameters comprising force impulse, peak force, mean force etc. 1005 (Fig. 10); and descent parameters comprising translation of fetal head (e.g. 1706 in Fig. 17), characteristics of translation of fetal head during a pull etc. Using data collected from obstetric extractions in preliminary studies, models that predict false descent due to elongation of fetal head are deduced by employing statistical, pattern-matching or similar techniques. Relevant parameters for these models are computed and accumulated 2602. These parameters include, but are not limited to, for example, the ratio between the translation of fetal head and the force impulse for a pull etc. The predictive models derived for the present algorithm are then used to calculate the probability of false descent 2603. If this probability remains above a prescribed limit (e.g. 80%) 2604, for a prescribed number of pulls (e.g. three pulls) 2605, haptic feedback is activated 2606 to notify the user and a warning with the probability value is displayed on the computer 2607. The user can thus make an overall assessment and may decide to end the obstetric extraction immediately (i.e. an early-exit). All prescribed values detailed herein are also deduced from preliminary studies. All relevant information/data is recorded to memory/database.

### END OF THE DETAILED DESCRIPTION OF FURTHER OPTIONAL ASPECTS OF EXEMPLARILY EMBODIMENTS.

Some embodiments of the presented subject-matter may allow the user, who may be a healthcare professional such as a physician or a midwife, to perform obstetric extractions with the apparatus 1 that may have enhanced functionality but does not significantly alter the standard procedure. The apparatus 1 may comprise various devices that provide effective decision-making support by measuring parameters related to an obstetric extraction, thus making the procedure safer to perform. Recording of all measurements may offers objective documentation of the procedure. Haptic feedback may let the user to focus on the procedure rather than being distracted by a computer screen and be promptly cautioned in critical situations.

A detailed description of exemplarily devices and algorithms (e.g., methods) of some embodiments has been give above. By pulling the proximal element 102, e.g., an instrumented handle, the user exerts traction force (also referred to as "extraction force" or simple "force" within the present specification, on the fetal head FH to pull it out of the birth canal mother.

Features of further embodiments are defined in the dependent claims. The features of further embodiments and the features of the embodiments described above may be combined with each other for forming additional embodiments, as long as the features are not explicitly described as being alternative to each other.

Spatially relative terms such as "under", "below", "lower", "over", "upper" and the like, are used for ease of description to explain the positioning of one element relative to a second element. These terms are intended to encompass different orientations of the respective device in addition to different orientations than those depicted in the figures. Further, terms such as "first", "second", and the like, are also used to describe various elements, regions, sections, etc. and are also not intended to be limiting. Like terms refer to like elements throughout the description.

As used herein, the terms "having", "containing", "including", "comprising", "exhibiting" and the like are open ended terms that indicate the presence of stated elements or features, but do not preclude additional elements or features. The articles "a", "an" and "the" are intended to include the plural as well as the singular, unless the context clearly indicates otherwise.

## Claims

1. An obstetric extraction apparatus (1), comprising:
- an extractor (10) having a distal element (101) and a proximal element (102) coupled thereto, the distal element (101) being configured to be docketed to a fetal head (FH), and the proximal element (102) being configured to be gripped by hand (HA);
- a beacon (11) configured to be attached stationary and separately from the extractor (10);
- a sensor (12) mechanically coupled to the extractor (10) and configured to provide a force signal representative of an extraction force applied to the fetal head (FH); and
- a processor (13) communicatively coupled to at least the extractor (10), wherein the processor (13) is configured to receive the force signal and to carry out a value-based determination of a progress parameter set indicative of at least one of a distance (d) between the extractor (10) and the beacon (11) and a motion of the extractor (10) relative to the beacon (11), and
a user feedback device (14) coupled to the processor (13),
wherein
- the user feedback device (14) comprises a haptic feedback unit (141) included in the proximal element (102).

2. The apparatus (1) of claim 1, wherein:
- the extractor (10) and the beacon (11) comprise transceiver means (101-1, 11-1) configured to transmit and/or receive a progress signal, the progress signal including at least one of an electromagnetic signal, a radar signal, an ultrasound signal and an inertial signal; and
- the processor (13) is configured to carry out the value-based determination of the progress parameter set in dependence of the progress signal.

3. The apparatus (1) of claim 1 or 2, wherein:
- the processor (13) is configured to carry out a value-based determination of a force parameter set in dependence of the force signal provided by the sensor (12).

4. The apparatus (1) of claim 3, wherein:
- the processor (13) is configured to control the user feedback device (14) in dependence of at least one of the determined force parameter set and the determined progress parameter set.

5. The apparatus (1) of one of the preceding claims, wherein:
- the sensor (12) comprises 3-axis-force-measurement means.

6. The apparatus (1) of claim 5, wherein the force signal provided by the sensor (12) includes a first force signal component indicative of an axial component of the applied extraction force and a second force signal component indicative of a bending component of the applied extraction force.

7. The apparatus (1) of one of the preceding claims, wherein:
- the processor (13) comprises storage means (131) storing a plurality of data sets, each data set being indicative of a force-pattern and/or a progress-pattern recorded for a previous real or virtual obstetric extraction; and
- the processor (13) is configured to compare at least one of the progress parameter set derived from the progress signal and the force parameter set derived from the force signal with each of the stored data sets.

8. The apparatus (1) of one of the preceding claims, wherein
- the processor (13) is configured to classify the current obstetric extraction as belonging to one of several categories and to control the user feedback device (14) based on the classification.

9. The apparatus (1) of claim 7, wherein
- the processor (13) is configured to predict the outcome of the current obstetric extraction in dependence of the comparison and to control the user feedback device (14) based on the prediction.

10. The apparatus (1) of claim 7 or 9, wherein the comparison includes applying at least of one of a Principal-Component-Analysis, a neural network and data mining.

11. The apparatus (1) of one of the preceding claims, wherein the processor (13) comprises a data logger (132), wherein the processor (13) is configured to log therein each of the progress parameter set and the force parameter set on a common time basis.

12. The apparatus (1) of one of the preceding claims, wherein the distal element (101) comprises a suction cup.

13. The apparatus (1) of one of the preceding claims, further comprising a head probe (15) coupled to the processor (13) and configured to be attached separately from the extractor (10), wherein the head probe (15) is configured to provide a first measurement signal to the processor (13), the first measurement signal being indicative of a descent of the fetal head (FH).

14. The apparatus (1) of one of the preceding claims, further comprising a skull sensor (17) coupled to the processor (13), wherein the skull sensor (17) is configured to provide a second measurement signal to the processor (13), the second measurement signal being indicative of a distance between a skull bone of the fetal head and the distal element (101) of the extractor (10).

## Patentansprüche

1. Geburtshilfeextraktionsvorrichtung (1), die Folgendes umfasst:
- einen Extraktor (10) mit einem distalen Element (101) und einem proximalen Element (102), die an ihn gekoppelt sind, wobei das distale Element (101) konfiguriert ist, an einen fötalen Kopf (FH) angedockt zu werden, und das proximale Element (102) konfiguriert ist, durch eine Hand (HA) ergriffen zu werden;
- eine Festpunktmarkierung (11), die konfiguriert ist, stationär und getrennt von dem Extraktor (10) angebracht zu werden;
- einen Sensor (12), der an den Extraktor (10) mechanisch gekoppelt ist und konfiguriert ist, ein Kraftsignal zu liefern, das für eine auf den fötalen Kopf (FH) angewandte Extraktionskraft repräsentativ ist; und
- einen Prozessor (13), der zumindest an den Extraktor (10) kommunikationstechnisch gekoppelt ist, wobei der Prozessor (13) konfiguriert ist, das Kraftsignal zu empfangen und eine wertbasierte Bestimmung einer Fortschrittsparametergruppe auszuführen, die einen Abstand (d) zwischen dem Extraktor (10) und der Festpunktmarkierung (11) und/oder eine Bewegung des Extraktors (10) in Bezug auf die Festpunktmarkierung (11) angibt, und eine Anwenderrückkopplungsvorrichtung (14), die an den Prozessor (13) gekoppelt ist,
wobei
- die Anwenderrückkopplungsvorrichtung (14) eine haptische Rückkopplungseinheit (141) umfasst, die in dem proximalen Element (102) enthalten ist.

2. Vorrichtung (1) nach Anspruch 1, wobei:
- der Extraktor (10) und die Festpunktmarkierung (11) Sendeempfängermittel (101-1, 11-1) umfassen, die konfiguriert sind, ein Fortschrittssignal zu senden und/oder zu empfangen, wobei das Fortschrittssignal ein elektromagnetisches Signal und/oder ein Radarsignal und/oder ein Ultraschallsignal und/oder ein Trägheitssignal enthält; und
- der Prozessor (13) konfiguriert ist, die wertbasierte Bestimmung der Fortschrittsparametergruppe in Abhängigkeit von dem Fortschrittssignal auszuführen.

3. Vorrichtung (1) nach Anspruch 1 oder 2, wobei:
- der Prozessor (13) konfiguriert ist, eine wertbasierte Bestimmung einer Kraftparametergruppe in Abhängigkeit von dem durch den Sensor (12) gelieferten Kraftsignal auszuführen.

4. Vorrichtung (1) nach Anspruch 3, wobei:
- der Prozessor (13) konfiguriert ist, die Anwenderrückkopplungsvorrichtung (14) in Abhängigkeit von der bestimmten Kraftparametergruppe und/oder der bestimmten Fortschrittsparametergruppe zu steuern.

5. Vorrichtung (1) nach einem der vorhergehenden Ansprüche, wobei:
- der Sensor (12) 3-Achsen-Kraftmessmittel umfasst.

6. Vorrichtung (1) nach Anspruch 5, wobei das durch den Sensor (12) gelieferte Kraftsignal eine erste Kraftsignalkomponente, die eine axiale Komponente der angewandten Extraktionskraft angibt, und eine zweite Kraftsignalkomponente, die eine Biegekomponente der angewandten Extraktionskraft angibt, enthält.

7. Vorrichtung (1) nach einem der vorhergehenden Ansprüche, wobei:
- der Prozessor (13) Speichermittel (131) umfasst, die mehrere Datengruppen speichern, wobei jede Datengruppe ein Kraftmuster und/oder ein Fortschrittsmuster angibt, das für eine vorherige reale oder virtuelle Geburtshilfeextraktion aufgezeichnet wurde; und
- der Prozessor (13) konfiguriert ist, die Fortschrittsparametergruppe, die von dem Fortschrittssignal abgeleitet ist, und/oder die Kraftparametergruppe, die von dem Kraftsignal abgeleitet ist, mit jeder der gespeicherten Datengruppen zu vergleichen.

8. Vorrichtung (1) nach einem der vorhergehenden Ansprüche, wobei
- der Prozessor (13) konfiguriert ist, die aktuelle Geburtshilfeextraktion als zu einer von mehreren Kategorien zugehörig einzuordnen und die Anwenderrückkopplungsvorrichtung (14) anhand der Einordnung zu steuern.

9. Vorrichtung (1) nach Anspruch 7, wobei
- der Prozessor (13) konfiguriert ist, das Ergebnis der aktuellen Geburtshilfeextraktion in Abhängigkeit von dem Vergleich vorherzusagen und die Anwenderrückkopplungsvorrichtung (14) anhand der Vorhersage zu steuern.

10. Vorrichtung (1) nach Anspruch 7 oder 9, wobei der Vergleich enthält, eine Hauptkomponentenanalyse und/oder ein neuronales Netz und/oder eine Datenbankauswertung anzuwenden.

11. Vorrichtung (1) nach einem der vorhergehenden Ansprüche, wobei der Prozessor (13) eine Datenprotokolleinrichtung (132) umfasst, wobei der Prozessor (13) konfiguriert ist, darin die Fortschrittsparametergruppe und die Kraftparametergruppe auf einer gemeinsamen Zeitbasis zu protokollieren.

12. Vorrichtung (1) nach einem der vorhergehenden Ansprüche, wobei das distale Element (101) eine Saugglocke umfasst.

13. Vorrichtung (1) nach einem der vorhergehenden Ansprüche, die ferner eine Kopfsonde (15), die an den Prozessor (13) gekoppelt ist und konfiguriert ist, getrennt von dem Extraktor (10) angebracht zu werden, umfasst, wobei die Kopfsonde (15) konfiguriert ist, ein erstes Messsignal an den Prozessor (13) zu liefern, wobei das erste Messsignal ein Absenken des fötalen Kopfes (FH) angibt.

14. Vorrichtung (1) nach einem der vorhergehenden Ansprüche, die ferner einen Schädelsensor (17), der an den Prozessor (13) gekoppelt ist, umfasst, wobei der Schädelsensor (17) konfiguriert ist, ein zweites Messsignal an den Prozessor (13) zu liefern, wobei das zweite Messsignal einen Abstand zwischen einem Schädelknochen des fötalen Kopfes und des distalen Elements (101) des Extraktors (10) angibt.

## Revendications

1. Appareil d'extraction obstétricale (1), comprenant :
un extracteur (10) ayant un élément distal (101) et un élément proximal (102) accouplé à celui-ci, l'élément distal (101) étant configuré pour venir épouser une tête fœtale (FH), et l'élément proximal (102) étant configuré pour être saisi à la main (HA) ;
une balise (11) configurée pour être fixée fixe et séparément de l'extracteur (10) ;
un capteur (12) accouplé mécaniquement à l'extracteur (10) et configuré pour fournir un signal de force représentatif d'une force d'extraction appliquée à la tête fœtale (FH) ;
un processeur (13) couplé en communication au moins à l'extracteur (10), le processeur (13) étant configuré pour recevoir le signal de force et pour réaliser une détermination, sur la base de valeurs, d'un ensemble de paramètres de progression indiquant une distance (d) entre l'extracteur (10) et la balise (11) ou un mouvement de l'extracteur (10) par rapport à la balise (11) ; et
un dispositif de rétroaction d'utilisateur (14) couplé au processeur (13), le dispositif de rétroaction d'utilisateur (14) comprenant une unité de réaction haptique (141) incluse dans l'élément proximal (102).

2. Appareil (1) selon la revendication 1, dans lequel :
l'extracteur (10) et la balise (11) comprennent un moyen émetteur-récepteur (101-1, 11-1) configuré pour émettre et/ou recevoir un signal de progression, le signal de progression incluant au moins un signal parmi un signal électromagnétique, un signal radar, un signal ultrasonore et un signal inertiel ; et
le processeur (13) est configuré pour réaliser la détermination, sur la base de valeurs, de l'ensemble de paramètres de progression en fonction du signal de progression.

3. Appareil (1) selon la revendication 1 ou 2, dans lequel :
le processeur (13) est configuré pour réaliser une détermination, sur la base de valeurs, d'un ensemble de paramètres de force en fonction du signal de force fourni par le capteur (12).

4. Appareil (1) selon la revendication 3, dans lequel :
le processeur (13) est configuré pour contrôler le dispositif de rétroaction d'utilisateur (14) en fonction de l'ensemble de paramètres de force déterminé et/ou de l'ensemble de paramètres de progression déterminé.

5. Appareil (1) selon l'une des revendications précédentes, dans lequel :
le capteur (12) comprend un moyen de mesure de force sur 3 axes.

6. Appareil (1) selon la revendication 5, dans lequel le signal de force fourni par le capteur (12) inclut une première composante de signal de force indiquant une composante axiale de la force d'extraction appliquée et une seconde composante de signal de force indiquant une composante de flexion de la force d'extraction appliquée.

7. Appareil (1) selon l'une des revendications précédentes, dans lequel :
le processeur (13) comprend un moyen de stockage (131) stockant une pluralité d'ensembles de données, chaque ensemble de données indiquant un modèle de force et/ou un modèle de progression pour une extraction obstétricale réelle ou virtuelle précédente ; et
le processeur (13) est configuré pour comparer l'ensemble de paramètres de progression déduit du signal de progression et/ou l'ensemble de paramètres de force déduit du signal de force à chacun des ensembles de données stockés.

8. Appareil (1) selon l'une des revendications précédentes, dans lequel :
le processeur (13) est configuré pour classer l'extraction obstétricale en cours comme appartenant à une de plusieurs catégories et pour contrôler le dispositif de réaction d'utilisateur (14) sur la base de la classification.

9. Appareil (1) selon la revendication 7, dans lequel :
le processeur (13) est configuré pour prédire le résultat de l'extraction obstétricale en cours en fonction de la comparaison et pour contrôler le dispositif de réaction d'utilisateur (14) sur la base de la prédiction.

10. Appareil (1) selon la revendication 7 ou 9, dans lequel la comparaison inclut l'application d'une analyse en composantes principales et/ou d'un réseau neuronal et/ou d'une exploration de données.

11. Appareil (1) selon l'une des revendications précédentes, dans lequel le processeur (13) comprend un enregistreur de données (132), le processeur (13) étant configuré pour y enregistrer chacun de l'ensemble de paramètres de progression et de l'ensemble de paramètres de force sur la base d'un temps commun.

12. Appareil (1) selon l'une des revendications précédentes, dans lequel l'élément distal (101) comprend une ventouse.

13. Appareil (1) selon l'une des revendications précédentes, comprenant en outre une sonde de tête (15) couplée au processeur (13) et configurée pour être fixée séparément de l'extracteur (10), la sonde de tête (15) étant configurée pour fournir un premier signal de mesure au processeur (13), le premier signal de mesure indiquant une descente de la tête fœtale (FH).

14. Appareil (1) selon l'une des revendications précédentes, comprenant en outre un capteur de crâne (17) couplé au processeur (13), le capteur de crâne (17) étant configuré pour fournir un second signal de mesure au processeur (13), le second signal de mesure indiquant une distance entre un os de crâne de la tête fœtale et l'élément distal (101) de l'extracteur (10).
